# NEUE EUROPÄISCHE PATENTSCHRIFT

(11) **EP 0 770 060 B2**
(45) Veröffentlichungstag und Bekanntmachung des Hinweises auf die Entscheidung über den Einspruch: **24.04.2002**
(45) Hinweis auf die Patenterteilung: 20.01.1999
(21) Anmeldenummer: 95925831.0
(22) Anmeldetag: 05.07.1995
(51) Int. Cl.: C07C 319/20, C07C 319/28, C07C 323/52, A23K 1/16

(54) **VERFAHREN ZUR HERSTELLUNG VON AMMONIUM-2-HYDROXY-4-(METHYLTHIO)-BUTYRAT**
PROCESS FOR PRODUCING AMMONIUM-2-HYDROXY-4-(METHYLTHIO)-BUTYRATE
PROCEDE DE PREPARATION D'AMMONIUM-2-HYDROXY-4-(METHYLTHIO)-BUTYRATE

(30) Priorität: 11.07.1994 DE 4424043
(43) Veröffentlichungstag der Anmeldung: 02.05.1997
(73) Patentinhaber: Degussa AG, 40474 Düsseldorf (DE)
(72) Erfinder: SUCHSLAND, Helmut, D-63517 Rodenbach (DE); KOHL, Heinz, D-63517 Rodenbach (DE)
(86) Internationale Anmeldenummer: EP9502600
(87) Internationale Veröffentlichungsnummer: WO9601808

(56) Entgegenhaltungen:
- GB-A- 915 193
- US-A- 2 745 745
- O. Favre-Bulle, Deklaration zum Experimental Report

## Beschreibung

Die Erfindung betrifft ein Verfahren zur Herstellung von Ammonium-2-hydroxy-4-methylthio-butyrat (MHAAS) durch Behandlung von 2-Hydroxy-4-methylthiobuttersäure (MHA) mit Ammoniak, wobei das MHA sowohl Monomeres als auch Dimeres und höhere Oligomere umfaßt und aus einem Reaktionsgemisch isoliert wird, welches durch Anlagerung von Blausäure (HCN) an Methylmercaptopropionaldehyd (MMP) und Hydrolyse mit HCl des dabei erhaltenen Methylmercaptopropionaldehyd-Cyanhydrins (MMP-CH) erhalten wird.

Nutrivitätsverbessernde Futtermittelzusatzstoffe sind heute ein unverzichtbarer Bestandteil der Tierernährung. Sie dienen der besseren Verwertung des Nahrungsangebotes, stimulieren das Wachstum und fördern die Eiweißbildung. Einer der wichtigsten dieser Zusatzstoffe ist die essentielle Aminosäure Methionin, die vor allem in der Geflügelaufzucht als Adjuvans eine herausragende Stellung einnimmt. Auf diesem Gebiet gewinnen aber zunehmend sog. Methionin-Ersatzstoffe an Bedeutung, da sie ähnliche wachstumsstimulierende Eigenschaften aufweisen wie die dafür bekannte Aminosäure.

Unter diesen ist die 2-Hydroxy-4-(methylthio)-buttersäure als Methionin-Hydroxy-Analoges, abgekürzt MHA, in racemischer Form als Additiv bekannt und wirtschaftlich bedeutend geworden.

MHA wird in Form wäßriger Lösungen mit Wirkstoffgehalten zwischen ca. 88 - 90 % gewonnen und eingesetzt. Allerdings enthalten diese Konzentrate nicht nur die monomere Säure, sondern infolge innermolekularer Veresterung auch Oligomere, hauptsächlich lineare und zyklische Dimere sowie in geringerem Umfang noch Tri- und Tetramere. Wegen des bekannt niedrigeren nutritiven Wirkungsgrades und der schlechteren Fließeigenschaften ist es wünschenswert, den Oligomerengehalt möglichst gering zu halten. Andererseits unterliegt die Bildung der Oligomeren wie bei allen α-Hydroxysäuren den Gesetzmäßigkeiten des chemischen Gleichgewichts, ihr Anteil ist somit nicht frei wählbar. Vielmehr hängt er ab von den die Gleichgewichtseinstellung bestimmenden Parametern wie Konzentration, Temperatur, pH-Wert und Wassergehalt. Zwar gelingt es aufgrund der Trägheit der Äquilibrierungsreaktion unter geeigneten Bedingungen Produkte mit deutlich niedrigerem Oligomerenanteil als dem Gleichgewicht entsprechend herzustellen; das Verhältnis von Monomeren zur Summe der Oligomeren liegt dann meist oberhalb ca. 4 : 1 und kann sogar Werte über 5 : 1 erreichen. Jedoch verschiebt sich das Verhältnis nach längerer Lagerung stetig zugunsten der Oligomeren, bis schließlich nach vollzogener Gleichgewichtseinstellung der Monomeranteil bis auf ca. 2,5 bis ca. 3 : 1 in Abhängigkeit von Wassergehalt, Temperatur und pH-Wert abgesunken ist Das Produkt "altert".

In diesem Alterungsprozeß liegt denn auch ein anwendungsspezifischer Nachteil des käuflichen MHA.

Als ein weiterer, produktionsspezifischer Nachteil schlägt der mit der Gewinnung des MHA verbundene Zwangsanfall von anorganischen Ammoniumsalzen zu Buche. So werden bei den heute in der Technik üblichen Herstellungsverfahren pro kg MHA etwa die gleiche Menge an Ammoniumsulfaten als Abwasserballast erzeugt (s.w.u.). Deren Entsorgung resp. Aufarbeitung zum Zwecke einer anderweitigen Verwertung ist aber mit Zusatzkosten verbunden, die den Prozeß und damit das Zielprodukt nicht unerheblich verteuern.

Zu den Methionin-Ersatzstoffen zählen auch bestimmte salzartige Verbindungen des MHA wie vor allem dessen Calziumsalz und gemischtes Calziumammoniumsalz. Diese haben aber nicht die gleiche kommerzielle Bedeutung erlangt wie die freie Säure, da ihre Herstellung mit höheren Gestehungskosten verbunden ist. Darüber hinaus lassen sie sich als pulverförmige, schwach hygroskopische Feststoffe nicht so einfach und homogen in die Futtermittelmischungen einbringen wie die leicht zu versprühenden wäßrigen Konzentrate der freien Säure.

Ein weiteres Additiv dieser Stoffklasse ist das Ammonium-2-hydroxy-4-(methylthio)-n-butyrat, abgekürzt MHAAS. Obgleich schon länger bekannt, hat MHAAS aber in der Tierernährung bisher keinen Eingang gefunden. Dies dürfte vor allem damit zusammenhängen, daß das Salz in reiner Form nur schwer und mit erheblichem Aufwand zu isolieren ist, wobei es entweder als viskoses Öl oder als zerfließliche und hygroskopische Masse erhalten wird. Auch weist die Handhabung eines solchen zur Inhomogenität neigenden Stoffes anwendungstechnische Probleme auf, weshalb es schwierig ist, seine biologische Wertigkeit exakt zu bestimmen. Jedenfalls liegen bislang noch keine gesicherten Erkenntnisse seiner Nutrivitätsäquivalenz gegenüber marktgängigen Produkten vor.

Ebenfalls bekannt als Methionin-Substitut ist das 2-Hydroxy-4-(methylthio)-n-butyramid, abgekürzt MHA-Amid. Allerdings gestaltet sich die gezielte Herstellung der Verbindung, welche als Zwischenstufe im MHA-Prozeß auftritt sowie die Abtrennung aus den Hydrolysegemischen sehr schwierig. Niedrige Ausbeuten des kristallinen MHA-Amids wie auch seine im Vergleich zu MHA selbst geringere nutritive Wirksamkeit sind denn auch die Gründe, weshalb dieser Stoff bislang kommerziell unbedeutend geblieben ist.

Das allgerneine Verfahren zur Herstellung von MHA und seiner AlkalilErdalkalisalze geht von 3-Methylmercaptoproprionaldehyd, auch als MMP bezeichnet, aus, den man mit Cyanwasserstoff zum 2-Hydroxy-4-methylthio-butyronitril, auch als MMP-Cyanhydrin oder MMP-CH bezeichnet, nitriliert (Gleichung I).

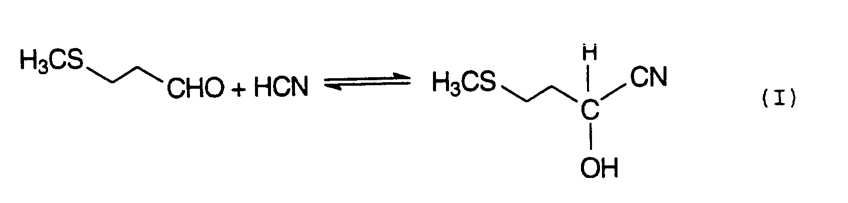

Das entstandene MMP-Cyanhydrin wird anschließend üblicherweise mit starken Mineralsäuren, wie H₂SO₄ oder HCI, über die Zwischenstufe des 2-Hydroxy-4-methylthiobutyramids, auch als MHA-Amid bezeichnet, (Gleichung II), 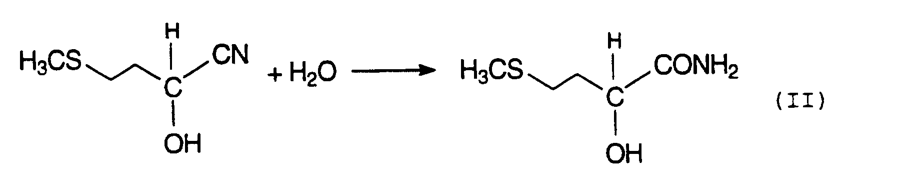 zum Methioninhydroxyanalogen (MHA) hydrolysiert (Gleichung III).

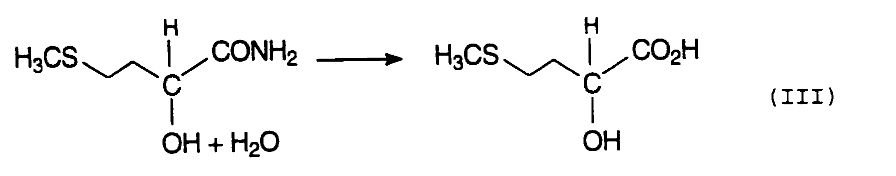

Diese Hydrolyse kann sowohl ein- als auch zweistufig durchgeführt werden. Um zum MHA-Salz zu gelangen wird das im Produktgemisch vorliegende MHA in geeigneter Weise, entweder direkt oder nach vorheriger Isolierung, behandelt.

Dabei können zur Isolierung so unterschiedliche Trenntechniken wie Lösungsmittelextraktion, Aussalzung, Fällung, Filtration und Aufkonzentrierung sowohl einzeln als auch in Kombination zur Anwendung kommen.

Die vorliegende MHA-Säure kann beispielsweise durch Behandlung mit Metalloxiden, -hydroxiden, -carbonaten zum gewünschten MHA-Salz weiterzuverarbeiten. Um zum Ammoniumsalz (MHAAS) zu gelangen kann man MHA z. B. mit Ammoniak behandeln (Gleichung IV):

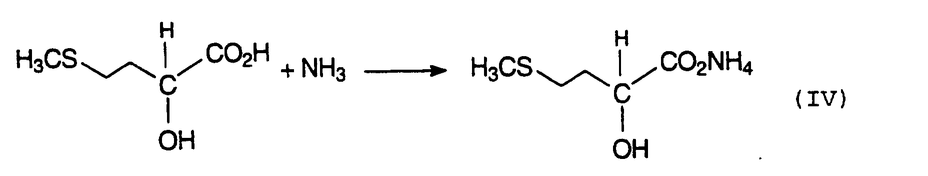

Dabei besteht in jedem Fall eine wichtige Aufgabe darin, das jeweilige Zielprodukt von den anorganischen Begleitund und Ballaststoffen abzutrennen, wobei deren Aufarbeitung nebst Verwertung bzw. Entsorgung nach heutigen Maßstäben ein wesentliches Problem darstellen und die Wirtschaftlichkeit des zugrunde liegenden Produktionsprozesses maßgeblich beeinflussen kann.

In der Patentliteratur sind eine Vielzahl von Verfahren beschrieben, die sowohl die Gewinnung des MHA als solches wie auch die der Alkali/Erdalkalisalze, vorzugsweise der Calzium- und gemischten Calziumammoniumsalze, zum Gegenstand haben.

Auf die Herstellung des MHA in Form wäßriger Konzentrate sind die Verfahren der europäischen Patentschriften EP-PS 142 488, 143 100, 330 521 und die der amerikanischen Schriften US-PS 3 773 927 und 4 353 924 gerichtet.

Eine zweistufige Hydrolyse liegt ausgehend von MMP-CH den Verfahren der EP-PS 142 488 (mit Schwefelsäure) und EP-PS 143 100 (mit Mineralsäure) zugrunde. Dort wird das Cyanhydrin zunächst bei relativ niedrigen Temperaturen mit z. B. 50 - 70%iger Schwefelsäure zum MHA-Amid hydratisiert, worauf nach Verdünnung mit Wasser die Hydrolyse bei höheren Temperaturen vervollständigt wird. Danach wird das Reaktionsgemisch extraktiv zerlegt, wobei bestimmte, mit Wasser partiell mischbare Lösungsmittel zur Anwendung kommen. Aus dem organischen Extrakt gewinnt man dann das MHA in konzentrierter wäßriger Lösung durch Verdampfung des Lösungsmittels unter festgelegten Bedingungen in Gegenwart von Wasser. Man erhält auf diese Weise mit sehr guter Ausbeute ein zunächst hochwertiges Endprodukt, das sich insbesondere durch geringe Verfärbung und einen vergleichsweise niedrigen Oligomerenanteil auszeichnet, welcher deutlich unter dem der Gleichgewichtszusammensetzung liegt. Nachteilig an dieser Verfahrensweise ist die Problematik des bei der produktzerlegung im wäßrigen Raffinat als Koppelprodukt abgeschiedenen Ammoniumbisulfats, über dessen Verbleib bzw. Entsorgung keine Angaben gemacht werden und das unbehandelt zu einer erheblichen, kaum vertretbaren Abwasserbelastung führt. Von Nachteil ist bei diesem Verfahren auch die vorerwähnte Alterungstendenz der im frischen Zustand mit vergleichsweise niedrigen, in jedem Fall aber unter dem GleichgewichtsverhältniS erhaltenen Monomer/Oligomer-Konzentrate.

MHA-Konzentrate ohne Zuhilfenahme eines Lösungsmittels gewinnt man nach US-PS 3 773 927 durch zweistufige Hydrolyse des MMP-CH mit Salzsäure, nachfolgende Aufkonzentrierung der Reaktionsmischung nebst Abtrennung des auskristallisierten Ammoniumchlorids. Das so erhaltene MHA ist jedoch stark verfärbt und reich an Oligomeren. Das als Coprodukt isolierte Ammoniumsalz ist ebenfalls verunreinigt, so daß es keine Verwertungschancen besitzt und somit als wertloser Ballaststoff anzusehen ist.

Eine Verbesserung des vorgenannten Verfahrens wird in der US-PS 4 353 924 dadurch erreicht, daß man die überschüssige Mineralsäure mit Ammoniak oder anderen alkalisch reagierenden Stoffen neutralisiert. Man erhält so MHA-Lösungen mit geringeren korrosiven Eigenschaften. Das Salzproblem aber bleibt bestehen.

In der EP-PS 330 521 wird schließlich noch ein einstufiges Hydrolyseverfahren mit Schwefelsäure beschrieben, das ohne Lösungsmittel auskommt, wobei neben flüssigem MHA kristallines Ammoniumsulfat als Coprodukt erhalten wird. Dieses Ziel wird erreicht, indem man die abreagierte Verseifungsmischung partiell mit Ammoniak neutralisiert, so daß das entstandene Ammoniumbisulfat ebenso wie noch vorhandene freie Schwefelsäure in das neutrale Anmoniumsulfat überführt werden. Dabei entstehen zwei flüssige Phasen, die ihrerseits getrennt und eingedampft werden, um einerseits flüssiges MHA und andererseits kristallines Ammoniumsulfat zu gewinnen. Durch geeignete Kombination der verschiedenen Filtrations- und Rückführungsschritte erreicht man, daß kein Produkt verloren geht und die Bildung eines mit Salzen belastetes Abwasser vermieden wird. Das resultierende MHA ist von sehr guter Qualität. Jedoch trotz dieser Vorteile weist das Verfahren auch erhebliche Nachteile auf. Zum einen ist das isolierte Ammoniumsulfat von klebriger Konsistenz und mit einem intensiven Geruch behaftet, so daß das Salz noch nachträglich beispielsweise durch Umkristallisation gereinigt werden muß, um es in verkaufsfähiger Form zu erhalten. Zum anderen entstehen durch die verdünntere Arbeitsweise in Verbindung mit dem dabei notwendigen höheren Mineralsäureüberschuß zusätzliche Kosten, die sich in höheren Verbrauchszahlen und einem größeren Energiebedarf z.B. bei den diversen Eindampfoperationen niederschlagen. Dazu kommt noch ein aufwendiges Feststoffhandling. Alle diese Faktoren verteuern das Verfahren in erheblichem Umfang. lm übrigen unterliegt das resultierende Monomer/Oligomer-Konzentrat dem gleichen Alterungsprozeß wie das nach EP-PS 142 488 resp. 143 100 erhaltene.

Auf die Herstellung der Alkali/Erdalkalisalze des MHA, insbesondere des Calzium- und gemischten Calziumammoniumsalzes, sind die Verfahren der Patentschriften US-PS 2 745 745, 2 938 053, 3 175 000, 4 310 690 und der GB-PS 722 024 sowie 915 193 gerichtet, wobei in allen Fällen der Salzbildung die Gewinnung des MHA-Intermediats durch mineralsaure Nitrilhydrolyse vorausgeht.

Die Zweistufenhydrolyse mit Schwefelsäure liegt den Verfahren der US-PS 2 745 745, 2 938 053 und 3 175 000 zugrunde. In den beiden zuerst genannten Schriften wird die verdünntere Verseifungsmischung mit z.B. Calziumhydroxid oder -carbonat in einer zumindest zur Bindung der Sulfationen ausreichenden Menge behandelt. Dabei wird Calziumsulfat ausgefällt und Ammoniak freigesetzt, der seinerseits mit MHA zum Ammoniumsalz MHAAS reagiert. Aus der Lösung kann das Salz durch Eindampfen in allerdings verunreinigter Form als viskoses Öl oder hygroskopischer Feststoff erhalten werden. Es kann aber auch vorzugsweise durch Zugabe von weiterem Calziumhydroxid/carbonat zum Calzium- oder Calziumammoniumsalz des MHA umgesetzt werden, welche nach dem Eindampfen in fester Form erhalten werden. Zum gleichen Ergebnis kommt man, wenn man die Verseifungslösung sofort mit einem ausreichenden Überschuß der basischen Calziumverbindung behandelt. In der GB-PS 722 024 wird als Ausgangsprodukt MHA-Amid verwendet. Im übrigen ist der Verfahrensweg zur Gewinnung der MHA-Salze mit dem oben beschriebenen identisch.

Um den Zwangsfall des wertlosen Calziumsulfat-Coproduktes zu vermeiden wird in der US-PS 3 175 000 die schwefelsaure Verseifungsmischung mit weiterem Ammoniumsulfat bis zur Sättigung versetzt. Aufgrund des Aussalzeffektes bilden sich zwei Phasen, wobei über 90 % des MHA in der organischen Phase abgeschieden werden. Dieses MHA kann unmittelbar zum Calziumsalz weiter verarbeitet werden. Aus der wäßrigen Phase wird das restliche MHA durch Lösungsmittelextraktion, vorzugsweise Ether, abgetrennt und entweder separat oder nach Vereinigung mit der Hauptfraktion unter Abdampfung des Lösungsmittels zurn Calziumsalz umgesetzt. Über den Verbleib des coproduzierten Ammoniumsulfats werden keine Angaben gemacht.

Eine Lösung der Ammoniumsalz-Problematik beschreibt das Verfahren der US-PS 4 310 690. Dort wird nach der zweistufigen Hydrolyse mit Salzsäure das Verseifungsgemisch mit Natronlauge unter definierten Bedingungen soweit neutralisiert, daß das gebildete Ammoniumchlorid vollständig in Natriumchlorid und Ammoniak zerlegt wird. Bei der anschließenden Behandlung mit stöchiometrischen Mengen an Ätzkalk erhält man das MHA-Calziumsalz als Aufschlämmung in einer praktisch gesättigten Kochsalzlösung. Nach der Fest/Flüssigtrennung wird ein Teil des Waschfiltrates für die Bereitung der Ätzkalk-Suspension zurückgeführt und man erhält als Abwasser eine Kochsalzlösung mit geringem organischen Ballast. Über die Verwendung bzw. den Verbleib des Coproduktes Ammoniak werden auch hier keine Angaben gemacht

Die GB-PS 915 193 schließlich beschreibt ein kontinuierliches Verfahren zur Gewinnung des Calzium- oder Ammoniumsalzes von MHA. Dem Verfahren liegt die einstufige Verseifung des MMP-Cyanhydrins mit stärker verdünnter, ca. 20%iger Schwefelsäure im Überschuß zugrunde. Aus dem nach 10stündiger Verweildauer kontinuierlich abgezogenen Verseifungsgemisch wird das MHA mit höher siedenden Ethern extrahiert, worauf durch nachfolgende Behandlung des Extrakts mit Calziumhydroxid/-carbonat-Aufschlämmungen in Wasser das MHA-Calziumsalz in feinkristalliner Form ausgefällt und abgetrennt wird. Leitet man zur Salzbildung gasförmigen Ammoniak in den etherischen MHA-Extrakt so bildet sich MHAAS, das in öliger Form abgeschieden wird. Die bei dem Verfahren vorgesehene Rückführung des wäßrig sauren Raffinats in die Hydrolysestufe führt allerdings zu einer Akkumulierung der Ammoniumsalze und läßt sich in der angegebenen Weise nicht durchführen. Darüberhinaus sind die langen Reaktionszeiten produktschädigend.

Angesichts des hierin diskutierten Standes der Technik ist es Aufgabe der vorliegenden Erfindung ein Verfahren zur Herstellung von Ammonium-2-hydroxy-4-(methylthio)-n-butyrat (MHAAS) anzugeben, welches die sowohl mit der Herstellung als auch mit der Anwendung bekannter Methionin-Ersatzstoffe einhergehenden, vorerwähnten Nachteile vermeidet oder zumindest merklich mindert. Dabei soll das Verfahren auch und vor allem unter dem Gesichtspunkt der Umweltverträglichkeit fortschrittlich sein, was insbesondere durch Vermeidung eines umweltbelastenden Abwassers bzw. Zwangsanfalls von anorganischen Ammoniumsalzen erreicht werden kann. Ein weiteres Ziel der Erfindung ist die Bereitstellung eines für die Tierernährung geeigneten, Wirkstoffs oder einer diesen Wirkstoff aufweisenden Wirkstoffmischung in flüssiger ünd stabiler Form, d.h. insbesondere mit gegenüber dem Stand der Technik erheblich verringerter bzw. ohne Alterungstendenz.

Gelöst werden diese und weitere nicht im einzelnen angegebene Aufgaben in verfahrensmäßiger Hinsicht durch ein Verfahren der eingangs erwähnten Art, mit den Merkmalen des kennzeichnenden Teils des Anspruchs 1.

Vorteilhafte Verfahrensausprägungen werden in den von Anspruch 1 abhängigen Ansprüchen unter Schutz gestellt.

Dadurch, daß das Reaktionsgemisch mit einem inerten, mit Wasser nicht oder nur teilweise mischbaren Lösungsmittel unter Erhalt eines ersten organischen Extrakts und eines ersten wäßrigen Raffinats behandelt wird, der erste organische Extrakt durch Behandlung mit Ammoniak unter Phasenseparierung in einen zweiten organischen Extrakt und ein zweites wässriges Raffinat zerlegt wird, wobei unter Salzbildung die Rückextraktion des MHA als MHAAS in das zweite wässrige Raffinat erfolgt, und das MHAAS aus dem zweiten wässrigen Raffinat isoliert wird, wird ein neuartiges Herstellverfahren für MHAAS und darauf basierende Wirkstoffmischungen bereitgestellt, das nur auf "flüssigen" Verfahrensschritten beruht und somit technisch vorteilhaft für die industrielle Fertigung geeignet ist. Zudem werden in bevorzugter Ausführungsform die Erzeugung eines mit Ammoniumsalzen belasteten Abwassers sowie die Co-Produktion von anorganischen Ballaststoffen wie Ammoniumchlorid und -sulfat oder Calziumsulfat vermieden, wobei ein im wesentlichen nicht mehr chemisch behandlungsbedürftiges Abwasser entsteht.

Durch die Anwendung des erfindungsgemäßen Verfahrens werden des weiteren ein Wirkstoff bzw. eine Wirkstoffmischung in flüssiger Form aus der Familie der Methionin-Ersatzstoffe mit verbesserten Eigenschaften bereitgestellt, die sich im Vergleich zu herkömmlichen Produkten dieser Gattung durch eine verbesserte Fließfähigkeit und durch einen erheblich verringerten bis gegen Null konvergierenden Oligomerengehalt auszeichnen.

Darüber hinaus sind die Wirkstoffe aufgrund ihrer schwach sauren bis neutralen Beschaffenheit keinen nennenswerten Alterungserscheinungen ausgesetzt, die bei bekannten Produkten von einem sich stetig ändernden, meist ansteigenden Oligomerenanteil herrühren und die sich ungünstig auf den biologischen Wirkungsgrad und das Fließverhalten auswirken können. Die Stoffe besitzen daher vor allem auch eine über eine längere Lagerzeit gleichbleibende und konstant hohe nutritive Wirksamkeit.

Ein wichtiger Aspekt des erfindungsgemäßen Verfahrens ist das Extrahieren der bei der Hydrolyse erhaltenen Lösung mit einem inerten mit Wasser nicht oder teilweise mischbaren Lösungsmittel unter Erhalt eines ersten organischen Extraktes mit dem darin überführten und gelösten MHA nebst Oligomeren sowie ggf. noch restlichem MHA-amid und eines wäßrigen, im wesentlichen Organik-freien ersten Raffinats in Form einer konzentrierten bis gesättigten Ammoniumsalzlösung.

Für die Extraktion sind alle polaren bis unpolaren, mit Wasser nicht oder nur teilweise mischbaren Lösungsmittel geeignet, die sich unter den Extraktionsbedingungen wie auch unter Bedingungen der Rückextraktion des folgenden Schrittes inert verhalten. Zu nennen sind sekundäre Alkohole, Ether, Ester, Ketone, aliphatische und aromatische Kohlenwasserstoffe, die außerdem chloriert sein können. Bevorzugte Lösungsmittel sind Ketone von C 5 - C 8, insbesondere Methylisobutylketon und Ethyl-n-Amylketon. Die Extraktion kann in jeder dem Fachmann geläufigen Weise vorgenommen werden, sofern gewährleistet ist, daß die Produktzerlegung praktisch vollständig erfolgt, d.h. daß das MHA, der Oligomeranteil sowie ggf. noch restliches MHA-amid weitestgehend in den Aufnehmer überführt werden; sie kann ansatzweise oder vorzugsweise kontinuierlich durchgeführt werden. Das Verhältnis von Produkt zu Aufnehmer ist nicht kritisch, jedoch sollte aus Gründen des Wirkungsgrades ein Mindestverhältnis von 1 : 1 nicht unterschritten und aus Gründe der Wirtschaftlichkeit ein Verhältnis von 1 : 3 nicht überschritten werden. Bevorzugt wird ein Verhältnis von 1 : 1,5 - 2,0.

Ein weiterer wichtiger Aspekt der Erfindung ist das Behandeln des ersten organischen Extraktes mit Ammoniak. Dies hat so zu geschehen, daß eine Aufspaltung des ersten organischen Extraktes in einer zweiten organischen Extrakt und ein zweites wäßriges Raffinats möglich wird. Dies kann unter Separierung der Phasen geschehen, wobei das angestrebte Produkt in die wäßrige Phase übergeht. Hierbei handelt es sich also um einen quasi zweiten Extraktionsschritt.

Menge und Konzentration des verwendeten Ammoniaks sind nicht kritisch, sofern gewährleistet ist, daß eine für die vollständige Salzbildung ausreichende Menge vorliegt. Aus Gründen der Wirtschaftlichkeit und Energieersparnis werden größere Überschüsse ebenso vermieden wie zu niedrige Konzentrationen. Bevorzugt wird ein hoch bis höchst konzentrierter Ammoniak. Ebenfalls nicht kritisch sind die in beiden Extraktionsschritten anzuwendenden Temperaturen, sofern das Phasentrennverhalten nicht beeinträchtigt und eine Emulsionsbildung vermieden wird. Bevorzugt werden Temperaturen von 20 - 60 °C. Höhere Temperaturen sind im allgemeinen weniger günstig, da es dann in der Regel zu unerwünschten Verdampfungserscheinungen kommt.

In bevorzugter Ausführungsform wird dabei der erste organische Extrakt so und in solcher Menge mit konzentrierter, vorzugsweise 25 - 90 gewichtsprozentiger wäßriger Ammoniaklösung versetzt, daß das darin enthaltene MHA im wesentlichen vollständig zum MHAAS neutralisiert wird. Bei der Neutralisation des sauren ersten organischen Extrakts werden auch Dimere und oligomere Anteile des MHA zu den entsprechenden Ammoniumsalzen umgewandelt. Der dabei erhaltene Extrakt enthält im wesentlichen das Extraktionslösungsmittel während das zweite Raffinat also die darin überführten Ammoniumsalze des MHA und seiner Oligomeren sowie ggf. noch restliches MHA-amid aufweist. In einem weiteren erfindungsgemäßen Aspekt ist es von Vorteil, das zweite wäßrige Raffinat vor und/oder während der Isolierung des angestrebten Produktes zu erhitzen, vorzugsweise unter Rückfluß. Dies ist auf zweierlei Weise günstig für das Verfahren der Erfindung. Zum einen werden die oligomeren Anteile sowie ggf. noch restliches MHA-amid weitgehend bis zu dem gewünschten Grenzgehalt unter Bildung von weiterem monomeren MHAAS gespalten und zum anderen kann eine im Rahmen der erfindungsgemäßen Zusammensetzung erwünschte geringe Menge an MHA durch hydrolytische Spaltung des MHAAS unter Freisetzen von Ammoniak rückgebildet werden.

Die "Rückextraktion" unter gleichzeitiger Neutralisation des MHA zu MHAAS, der Oligomeren zu NH₄-Oligomeren mittels Ammoniak wird durch die Tatsache ermöglicht, daß die genannten Salze in den Extraktionslösungsmitteln praktisch unlöslich sind und auch ggf. noch in geringer Menge vorhandenes MHA-Amid dank seines hydrophilen Charakters leicht in die wäßrige Phase übergeht.

In bevorzugter Verfahrensvariante wird aus dem zweiten Raffinat ggf. nach Oligomerenspaltung und Rückbildung einer gewünschten Menge MHA ggf. überschüssiger Ammoniak abgedampft in Verbindung mit ggf. einer Aufkonzentrierung durch Wasserverdampfung mittels einer adiabatischen Vakuum-Verdampfungskühlung unter Erhalt der angestrebten Wirkstofflösung;

In diesem Schritt kann die die Zielprodukte enthaltende Lösung so konditioniert werden, daß ein Wirkstoffkonzentrat der erfindungsgemäßen Zusammensetzung erhalten wird. Dieses Ziel erreicht man bevorzugt, indem der ggf. überschüssige Ammoniak abgetrieben, die Lösung ggf. durch weitere Wasserverdampfung bis zu der gewünschten Endkonzentration eingeengt wird, die Lösung unter Mitwirkung einer adiabatischen Vakuum-Verdampfungskühlung auf Temperaturen unter ca. 60 °C abgekühlt und die resultierende Lösung ggf. durch Zugabe von Ammoniak und/oder Wasser auf den gewünschten pH-Wert eingestellt wird.

Aufgrund der Variabilität der die Wirkstofflösung bildenden Verfahrensschritte ist es erfindungsgemäß möglich, eine den klimatisch wie technisch bedingten Lagerungsbedingungen optimal angepaßte Wirkstofflösung innerhalb der erfindungsgemäßen Grenzen bereitzustellen. So wird man z.B. für heißere Klimazonen ein Konzentrat bereitstellen, das einen etwas höheren, jedoch maximal 20 % nicht übersteigenden Anteil an freiem MHA besitzt mit entsprechend erniedrigtem pH-Wert, um so der möglichen Hydrolyse des MHAAS bei höheren Temperaturen entgegenzuwirken. Da der nutritive Wirkungsgrad von oligomerenreichen Lösungen vorteilhaft von demjenigen der reinen MHAAS-Lösung nicht gravierend abweicht, jedoch sich deutlich von dem MHA- und oligomerenreicher Lösungen absetzt, hat eine solche, die Lagerstabilität unterstützende Maßnahme keine Qualitätseinbuße zur Folge.

Durch die Verwendung von HCl wird ein verbessertes Hydrolysierverfahren bereitgestellt, das im Vergleich zu den gattungsgemäß bekannten Verfahren nach dem Stand der Technik die Gewinnung der MHA-Zwischenstufe in höherer Ausbeute, Reinheit, in geringerer Verfärbung und unter weniger Nebenproduktbildung sowie im wesentlichen frei von (geruchsintensiven) organischen Begleitstoffen gestattet. In vorteilhafter Ausgestaltung des erfindungsgemäßen Verfahrens wird die Hydrolyse des MMP-CH in zwei Stufen durchgeführt, wobei in einer ersten Stufe mit wäßriger Salzsäure mit einer Konzentration von 15 - 40 %, vorzugsweise 20 - 37 % und besonders bevorzugt von 30 - 36 % in einem Temperaturbereich von 20 - 70 °C, vorzugsweise von 30 - 60 °C, und besonders bevorzugt von 35 - 55 °C, sowie einem Molverhältnis MMP-CH zu HCI von 1 : 1,0 - 1,5, vorzugsweise von 1 : 1,05 - 1,3 und besonders bevorzugt von 1 : 1,1 - 1,2 hydrolisiert wird. Hierbei entsteht im wesentlichen MHA-amid und die Reaktionsmischung ist Cyanhydrin-frei. Weiterhin von ganz besonderem Vorteil ist das Hydrolysieren der Reaktionsmischung in einer zweiten Stufe, ggf. nach vorheriger Verdünnung mit Wasser, bei Temperaturen von 80 - 110 °C vorzugsweise 90 - 100 °C und besonders bevorzugt 90 - 95 °C, um die Hydrolyse des MHA-amids zum MHA im wesentlichen zu vervollständigen; anschließend wird das Hydrolysat günstigerweise unter Anwendung einer adiabatischen Vakuum-Verdampfungskühlung der Reaktionslösung auf mindestens 60 °C gekühlt, um eine geringe Menge flüchtiger Verunreinigungen sowie überschüssige Salzsäure zu entfernen.

Mit Vorteil werden beide Hydrolysestufen der zweistufigen Hydrolyse mit höher konzentrierter Salzsäure durchgeführt, da hiermit einerseits größere Säureüberschüsse vermieden werden und andererseits die Reaktion unter schonenden Bedingungen vonstatten geht. Beispielsweise erfordert eine 37 bis geringstenfalls ca. 30%ige HCl einen molaren Überschuß von nur noch 5 - bis maximal 15 %, während die zugehörigen Reaktionstemperaturen und -zeiten auf 30 - 50 °C/10 - 30 Minuten in Schritt (1) und 90 - 95 °C/45 - 60 Minuten in Schritt (2) begrenzt werden können.

Die sich anschließende adiabatische Verdarnpfungskühlung dient sowohl der energiesparenden Abkühlung als auch dem Entfernen geringer flüchtiger Verunreinigungen aus der Reaktionsmischung, welche in Letztere ggf. bereits mit dem Edukt eingeschleppt werden können (HCN, MMP). Auch das Entfernen eines Teils des Salzsäure-Überschusses kann von Vorteil sein. Wobei auf diesen Schritt ggf. auch verzichtet werden kann.

Weiterhin ist es in bevorzugter Verfahrensmodifikation günstig, das Reaktionsgemisch nach Hydrolyse und vor Behandlung mit Lösungsmittel mit einer Base zu behandeln.

Dabei dient die Base zum Abstumpfen oder Vorneutralisieren der überschüssigen freien Mineralsäure, die bis zum Abstumpfen für einen pH-Wert von ca. -1 oder noch stärker sauer sorgt.

Bevorzugt wird die Lösung mit wässrigem oder vorzugsweise gasförmigem Ammoniak zur Neutralisation der verbliebenen ggf. überschüssigen Salzsäure unter Einstellung eines pH-Wertes im Bereich von -0,5 bis +0,5, vorzugsweise von -0,2 bis +0,2, unter Erhalt einer wäßrigen Lösung, die im wesentlichen MHA nebst anteiligen Oligomeren sowie Ammoniumchlorid enthält, abgestumpft.

Die partielle Neutralisation der Reaktionsmischung mit (vorzugsweise gasförmigem) Ammoniak zum Abstumpfen der überschüssigen Mineralsäure verhindert eine Anreicherung der letzteren bei der nachfolgenden extraktiven Produktzerlegung und damit letztlich eine Anreicherung von Ammoniumsalzen im Zielprodukt.

In einem weiteren Aspekt ist es für die Erfindung bevorzugt, die im Prozeß auftretenden Stoffe möglichst im Kreislauf zu führen, wiederzuverwenden oder in eine gefahrlos zu entsorgende Form zu überführen.

So wird bei Verwendung von Salzsäure zur Hydrolyse das im wesentlichen Ammoniumchlorid enthaltende erste Raffinat vorteilhaft mit Natronlauge bis zu pH-Werten von mindestens 8 unter Freisetzung von Ammoniak und Bildung einer mit Ammoniak gesättigten Natriumchloridlösung neutralisiert. Bevorzugt ist auch das Abdestillieren und Aufkonzentrieren des in verschiedenen Schritten des Verfahrens erhaltenen Ammoniaks in einer oder mehreren Stufen. Mit und ohne Anwendung von Druck und ggf. rektifizierenden Bedingungen wird einerseits eine hoch bis höchst konzentrierte wäßrige Ammoniaklösung erhalten, die in die Behandlung des ersten organischen Extrakts zurückgeführt wird. Andererseits wird eine im wesentlichen ammoniakfreie, kochsalzhaltige Lösung erhalten, die einer biologischen Abwasseraufbereitung zugeführt werden kann. Die angesprochene Neutralisationsbehandlung mit NaOH, bei welcher der für die MHA-Konvertierung zum MHAAS benötigte Ammoniak, der hauptsächlich aus dem Nitrilstickstoff des Edukts gebildet wird, zurückgewonnen wird, wird bevorzugt mit konzentrierter Natronlauge ausgeführt.

Diese Neutralisationsbehandlung kann sowohl getrennt als auch in Kombination mit folgenden Destillations- und Aufkonzentrierungsschritten erfolgen. In beiden Fällen kommen die dem Fachmann bekannten Techniken und geeignete Apparaturen zur Anwendung. Dies gilt auch für die damit einhergehende Aufkonzentrierung, die in einem oder mehreren Schritten und in Abhängigkeit von der gewünschten Konzentration mit oder ohne Anwendung von Druck nach in der Technik bekannten Verfahren durchgeführt werden kann.

Die bei der MHA-Konvertierung und Rückextraktion als zweiter organischer Extrakt erhaltene Lösungsmittelphase wird vorteilhaft zur Vermeidung der Anreicherung von Verunreinigungen teilweise oder zur Gänze einer Reinigungsdestillation unterzogen, wobei je nach Art des verwendeten Lösungsmittels und des Anteils gelösten Wassers diese in Form einer Strippdestillation oder unter azeotropen und/oder rektifizierenden Bedingungen durchgeführt werden kann. Auf diesen Schritt kann ggf. intermittierend oder auch ganz verzichtet werden.

Von besonderem Vorteil für die Erfindung kann es sein, die verschiedenen Verfahrensschritte intermittierend oder kontinuierlich oder auch miteinander kombiniert durchzuführen. So kann es von Vorteil sein, die Reaktionsschritte ansatzweise und die Aufarbeitungsschritte kontinuierlich vorzunehmen. Selbstverständlich ist aber auch eine nur ansatzweise oder nur vollkontinuierliche Herstellung und Aufarbeitung möglich. Eine zumindest teilkontinuierliche Arbeitsweise ermöglicht die Anwendung schonender Verfahrensbedingungen und eine geringere Produktbelastung.

Von besonderem Vorteil gegenüber den gattungsgemäß zuzuordnenden bekannten Verfahren ist noch, daß im erfindungsgemäßen Verfahren keine Maßnahmen zur Unterdrückung bzw. Hintanhaltung der Oligomeren zu treffen sind, da die Oligomeren hier weitgehend aufgespalten werden. Ein weiterer Vorteil des Verfahrens der Erfindung liegt in dem Wegfall eines Feststoffhandlings, da kein Abfallsalz produziert wird und der bei der Hydrolyse gebildete Ammoniumstickstoff unter Salzbildung ohne Beeinträchtigung der Wirkstoffeigenschaften in dem Wirkstoff verbleibt.

Im Rahmen der vorliegenden Erfindung wird also ein neues Verfahren für diese Wirkstofformulierung bereitgestellt, das kein behandlungsbedürftiges ammoniumsalzhaltiges Abwasser und/oder kristalline Ammoniumsalze als Koppelprodukte erzeugt, das nur aus flüssigen Verfahrensschritten besteht und deshalb einfach und wirtschaftlich durchzuführen ist und das die Herstellung des Zielproduktes im gattungsgemäß bekannten Verfahrensteil der Hydrolyse unter verbesserten Herstellbedingungen mit verbesserter Produktqualität und geringerer Nebenproduktbildung gestattet sowie auch für die Gewinnung des Endproduktes keine weiteren Hilfschemikalien mit Ausnahme der für die Produktzerlegung anzuwendenden Natronlauge sowie des Extraktionslösungsmittels benötigt, wodurch das Verfahren verteuert würde.

Die in den Einzelschritten beschriebene Verfahrensweise läßt naturgemäß eine Vielzahl von Gestaltungsmöglichkeiten zu. Jedoch wird man optionell solche Parameter und Bedingungen wählen, die sowohl eine einfache und billige Verfahrensdurchführung gestatten als auch zu hervorragenden Ergebnissen bezüglich Ausbeute und Produktreinheit führt.

Durch die Art der Herstellung und Isolierung ist weiterhin vorteilhaft die Gewinnung eines ca. 80 - 90%igen Endproduktes in Form seines Ammoniumsalzes mit untergeordneten Beimengungen an freiem MHA nebst ggf. dessen Amid mit äußerst geringer Verfärbung, guter Fließfähigkeit, ausreichender thermischer Stabilität mit einem vorbestimmbar niedrigen bis gegen Null konvergierenden (falls gewünscht) Oligomerenanteil, der vorzugsweise bei 0,1 bis 2,0 % liegt, so daß eine Wirkstoffkombination entsteht, die im Vergleich zu den bekannten Produkten nicht mehr altern kann und deshalb nicht die mit dem Handling und der Lagerung einhergehenden Nachteile und Äquivalenzunsicherheiten aufweist.

Ein Produkt des Verfahrens ist daher auch ein Ammonium-2-hydroxy-4-methylthio-n-butyrat (MHAAS) in flüssiger Form mit einem Gesamtgehalt an MHAAS umfassend dimere und oligomere Anteile von 70 - 90 Gewichtsprozent, vorzugsweise 80 - 90 Gewichtsprozent und besonders bevorzugt 84 - 88 Gewichtsprozent.

Das erzeugte MHAAS ist gekennzeichnet durch einen zeitlich wenig bis nicht veränderlichen Anteil an Dimeren und Oligomeren von ≤ 10 Gewichtsprozent vorzugsweise ≤ 6 Gewichtsprozent und ganz besonders bevorzugt ≤ 1 Gewichtsprozent jeweils bezogen auf die Summe MHAAS + Dimer + Oligomer.

Zu den Produkten des Verfahrens gehören auch Mischungen aus Ammonium-2-hydroxy-4-methylthio-n-butyrat (MHAAS) mit 2-Hydroxy-4-(methylthio)-n-buttersäure (MHA) und/oder 2-Hydroxy-4-(methylthio)-n-butyramid (MHA-Amid).

Diese Mischungen weisen bevorzugt einen Anteil an MHA von bis zu 20 Gewichtsprozent und/oder an MHA-Amid bis zu 5 Gewichtsprozent, jeweils bezogen auf die Summe von MHAAS + MHA + MHA-Amid auf.

In einer weiteren Ausführungsform sind die Mischungen durch einen zeitlich wenig bis nicht veränderlichen Anteil an Dimeren and Oligomeren des MHAAS, MHA und/oder MHA-Amid von ≤ 10, vorzugsweise ≤ 6 und ganz besonders bevorzugt ≤ 1 Gewichtsprozent bezogen auf die Summe MHAAS + MHA + MHA-Amid + deren Dimere und Oligomere gekennzeichnet.

Nach dem erfindungsgemäßen Verfahren gewonnene MHAAS als Hauptbestandteil aufweisende Lösungen haben außerdem bevorzugt einen pH-Wert im lagerstabilen Zustand von 3,0 bis 7,5, vorzugsweise 4,0 - 7,0 und besonders bevorzugt 5,0 - 6,0. Sie zeichnen sich weiterhin aus durch äußerst geringe Verfärbung, gute Fließfähigkeit und befriedigende thermische Stabilität.

Nach der Erfindung erhältliche MHAAS als Hauptbestandteil und ggf. MHA und/oder MHA-Amid als untergeordnete Nebenbestandteile enthaltende Wirkstoffkonzentrate sind hervorragende Futtermittelzusatzstoffe mit nutrivitätsverbessernden und wachstumfördernde Eigenschaften.

Nachfolgend wird die Erfindung an Hand von Beispielen unter Bezugnahme auf die beigefügten Zeichnungen weitergehend erläutert. Soweit nicht anders angegeben, beziehen sich Prozentangaben auf Gew.-%.

In den Zeichnungen zeigen:
- Figur 1: eine schematische Darstellung einer Anlage zur Durchführung des erfindungsgemäßen Verfahrens;
- Figur 2: eine schematische Darstellung einer anderen Ausführungsform einer Anlage zur Durchführung einer Modifikation des erfindungsgemäßen Verfahrens; und
- Figur 3: ein Fließdiagramm für den schematischen Ablauf des erfindungsgemäßen Verfahrens;
- Figur 4: Abbildung oben:
zur Verdeutlichung des Einflusses verschiedener Methioninquellen auf die Performance von 7 - 28 Tage alten Broilern eine Auftragung des Gewichtszuwachses in (g) von Broilern gegen die supplementierten Methionin-Equivalente (%) für verschiedene Methioninquellen;
Abbildung unten:
zum selben Zweck wie oben eine Auftragung der Futterverwertung von Broilern gegen die supplementierten Methionin-Equivalente in (%) für verschiedene Methioninquellen; und

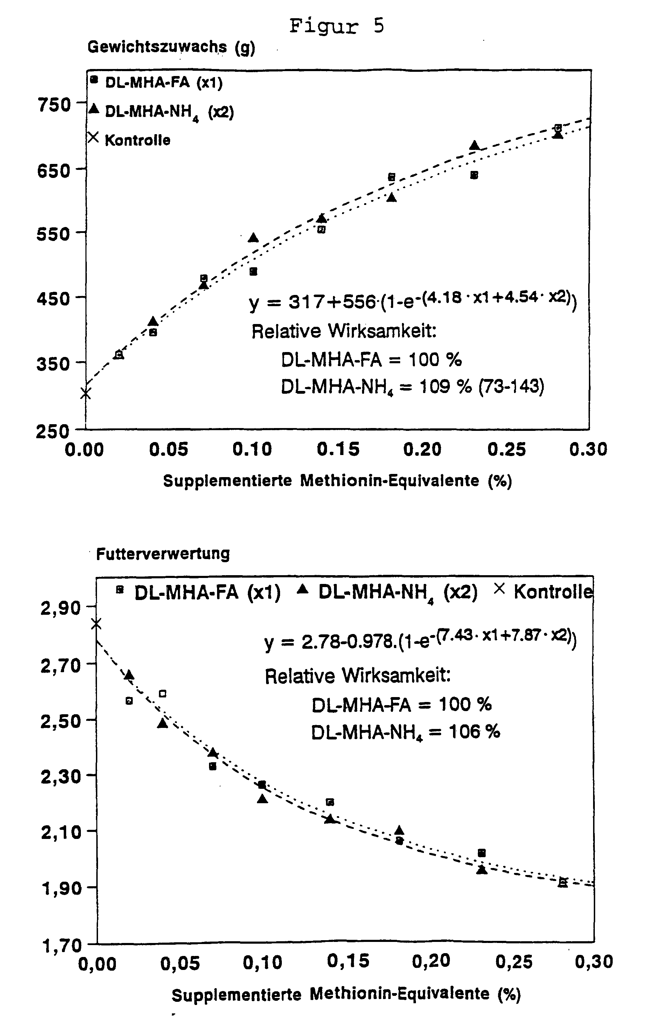

- Figur 5: Abbildung oben:
zur Verdeutlichung der nutritiven Wirksamkeit von DL-MHA (freie Säure), DL-MHA (Ammoniumsalz) und DL-Methionin eine graphische Darstellung des Gewichtszuwachses von Broilern in (g) gegen die supplementierten Methionin-Equivalente (%) für die verschiedenen Methioninquellen;
sowie
Abbildung unten:
zum selben Zweck wie oben eine graphische Darstellung der Futterverwertung von Broilern gegen die supplementierten Methionin-Equivalente (%) für verschiedene Methioninquellen.

### Beispiel 1

In einem mit Intensivrührer und Rückflußkühler ausgestatteten Reaktionsgefäß wurden 103,6 g 37%ige Salzsäure (1,05 Mol) bei 38 - 40 °C vorgelegt. Innerhalb 30 Minuten wurden bei dieser Temperatur unter intensivem Rühren und Kühlen 132,5 g 99,0%iges MMP-CH (1,0 Mol), welches nach bekannten Verfahren bereitet worden war, zugegeben. Unter Konstanthaltung der Temperaturen ließ man die Mischung unter Turbinieren 15 Minuten nachreagieren, worauf mittels HPLC-Analyse die vollständige Umsetzung des Cyanhydrins festgestellt wurde. Dabei waren ca. 90 % des Edukts zum MHA-amid und ca. 10 % zum MHA umgesetzt worden. Anschließend wurde der Reaktorinhalt mit 50 g Wasser verdünnt, auf 90/91 °C aufgeheizt und 45 Minuten lang gerührt. Nachdem die Vollständigkeit der Hydrolyse der Amidstufe zur freien Säure mittels HPLC überprüft worden war, wurde die Reaktionsmischung unter Zuhilfenahme einer Vakuum-Verdampfungskühlung bis auf ca. 40 °C abgekühlt, wobei geringe Mengen flüchtiger Bestandteile und Verunreinigungen (u.a. HCN) abgetrieben wurden. Die resultierende, schwach verfärbte und nahezu geruchsfreie Verseifungslösung mit einen pH-Wert um -0,9 wurde zur Bindung des Salzsäureüberschusses mit ca. 5%iger Ammoniaklösung bis auf einen pH-Wert von +0,1 abgestumpft und danach mit zweimal 250 ml Methylisobutylketon extrahiert. Die vereinigten Extrakte mit einem Gehalt von ca. 27% MHA-gesamt, wovon ca. 2,16 % in oligomerer, hauptsächlich dimerer Form vorlagen, wurden anschließend mit 50 g Wasser versetzt und danach mit 88,5 g einer 25%igen Ammoniaklösung (1,3 Mol) ausgeschüttelt. Dabei wurden alle MHA-Spezies in deren Ammoniumsalze (MHAAS, NH₄-Oligomere) umgewandelt, die ebenso wie noch restliches MHA-amid praktisch vollständig in die wäßrig/ammoniakalische Phase eluiert wurden. Das Eluat, die abgetrennte Unterphase, wurde nun 35 Minuten am Rückfluß erhitzt, danach unter Entfernung des überschüssigen Ammoniaks sowie noch vorhandener Lösungsmittelreste bis auf eine Konzentration von um 85 - 86 MHA-gesamt eingeengt. Es wurden 189,8 g Lösung mit 85,0 % MHAAS (monomer), 0,44 % MHA-amid, 0,16 % NH₄-Dimere sowie 15% Wasser erhalten. Die Gesamtausbeute betrug 97,1 % bezogen auf eingesetztes MMP-CH. Die Lösung war fahlgelb gefärbt, geruchsfrei, freifließend und mit einem pH-Wert von 6,5 bei Normaltemperaturen monatelang ohne Veränderungen lagerstabil.

Die nach der Rückextraktion erhaltene MIBK-Phase enthielt < 0,03 % an MHAAS und ca. 0,1 % an deren organischen Verunreinigungen (MHA-Oligomere). Sie wurde zwecks Reinigung des Lösungsmittels einer Stripp-Destillation unterworfen. Das regenerierte MIBK wurde nach Verlustergänzung für nachfolgende Ansätze vorgehalten. Das Sumpfprodukt wurde verworfen oder entsorgt.

Die aus der ersten Extraktion resultierende wäßrige Phase, welche im wesentlichen aus einer gesättigten Ammoniumchloridlösung bestand, das sog. Primär-Raffinat, wurde in eine beheizte Rührwerksapparatur mit Zulauftrichter M & R-Einrichtungen für Temperatur und pH sowie Rückflußkühler mit aufgesetztem Dephlegmator nebst einer angeschlossenen, auf bis -5 °C gekühlten Absorptionsvorlage überführt. Das Raffinat wurde unter stetiger Erwärmung durch Zugabe von konzentrierter Natronlauge bis zu einem pH-Wert von mindestens 8,5 neutralisiert. Der dabei freigesetzte Ammoniak wurde in der mit Wasser beschickten Vorlage absorbiert und in Form einer 20 - 25%igen Lösung zurückgewonnen und somit für weitere Folgeansätze bereitgestellt. Als Sumpfprodukt wurde eine im wesentlichen Organik-freie, mit Natriumchlorid gesättigte Abwasserlösung erhalten, die als solche nicht mehr behandlungsbedürftig war und verworfen bzw. entsorgt wurde.

### Beispiele 2 - 5

Es wurde wie in Beispiel 1 beschrieben verfahren, jedoch wurden für die extraktive Produktzerlegung anstelle des MIBK nacheinander folgende Lösungsmittel verwandt: Methylisopropylketon, Ethyl-n-amylketon, Methyl-t-butylether und Toluol. Bei prinzipiell gleicher Aufarbeitungsweise wurden ähnliche Ergebnis erzielt, wobei für die Konvertierung und Eluierung des MHA-gesamt zu deren Ammoniumsalzen jeweils die zurückgewonnenen 20 - 25%ige Ammoniaklösungen nach entsprechender Verlustergänzung des vorausgehenden Ansatzes eingesetzt wurden. Im Falle des Toluols wurde viermal mit je 250 ml extrahiert, wobei aber auf eine destillative Reinigung des Lösungsmittels für einen etwaigen Folgeansatz verzichtet werden konnte. Im Falle des Methyl-t-butylethers wurde das Hydrolysat vor der Extraktion auf ca. 20 °C abgekühlt.

In allen Fällen wurden nach der Konditionierung hell gefärbte, geruchsfreie und freifließende Lösungen mit Gehalten von 84 - 86 % an MHAAS-gesamt erhalten. Der Anteil an NH4-Oligomeren (Dimeren) überstieg in keinem Fall 0,2 %, der Gehalt an MHA-amid ließ sich auf unter 1,2 % begrenzen (bezogen auf MHAAS-gesamt).

### Beispiel 6

In einer wie im Beispiel 1 beschriebenen Reaktionsapparatur wurden 108,5 g 37%ige Salzsäure (1,1 Mol) bei 30 °C vorgelegt. Innerhalb von 30 Minuten wurden bei dieser Temperatur unter intensivem Rühren und Kühlen 132,5 g 99%iges MMP-CH (1,0 Mol) zugegeben. Nach weiteren 10 Minuten Rührdauer war die Umsetzung des Cyanhydrins vollständig und dieses analytisch nicht mehr nachweisbar. Der Reaktorinhalt wurde anschließend in ca. 15 Minuten auf 90 °C erhitzt und bei dieser Temperatur weitere 30 Minuten turbiniert. Danach wurde auf ca. 40 °C ohne Zuhilfenahme einer Verdampfungskühlung abgekühlt. Das hierbei auskristallisierte Ammoniumchlorid wurde durch Zugabe von 50 g Wasser wieder in Lösung gebracht. Es wurden 302,5 g einer praktisch farblosen Lösung von schwachem, aber angenehmen Geruch mit folgender Zusammensetzung erhalten:

43,80 % MHA-monomer, 5,46 % MHA-dimer + -trimer, 0,17 % MHA-amid, 17,52 % Ammoniumchlorid und 33,05 % Wasser. Das Verhältnis von Monomerem zu Oligomeren betrug 8,03 : 1 mit 11,07 % Oligomeren am Gesamt-MHA. Die Gesamtausbeute an MHA + Oligomeren + Amid betrug 99,5 % bezogen auf eingesetztes MMP-CH. Der pH-Wert lag bei -1,0.

Die Lösung wurde zweimal mit je 300 ml MIBK extrahiert. Die vereinigten Extrakte wurden zur Entfernung der anteilig gelösten Salzsäure bis auf 60 Vol% eingeengt, wobei die Hauptmenge in Form eines ternären Azeotrops abdestilliert wurde. Das in einem Abscheider aufgefangene zweiphasige Brüdenkondensat wurde zur Neutralisation der Salzsäure mit verdünnter Ammoniaklösung behandelt. Nach der Phasentrennung wurde die obere Phase mit dem Extraktkonzentrat vereinigt während die untere Phase dem Primär-Raffinat zugeschlagen wurde. Die vereinigten Extrakte mit einem Gehalt von ca. 26 % MHA-gesamt wurden mit 102 g 25%iger Ammoniaklösung (1,5 Mol) ausgeschüttelt wobei das MHA inklusive Oligomere und Restamid praktisch vollständig in die wäßrige Phase, erstere als Ammoniumsalze, eluiert worden waren. Das Eluat wurde 25 Minuten zum Rückfluß erhitzt und danach unter Entfernung des überschüssigen Ammoniaks eingeengt. Es wurden 193,5 g einer fahlgelben Lösung der folgenden Zusammensetzung erhalten:

85,2 % MHAAS, 0,69 % MHA-amid, 0,09 % Dimere, 14,0 % H₂O sowie 0,1 % Cl⁻. Die Lösung hatte einen pH-Wert von 6,8. Sie war von befriedigender Lagerstabilität, freifließend und praktisch geruchsfrei.

Das Primär-Raffinat wurde mit Ammoniak-haltigen Kondensaten vereinigt und wie in Beispiel 1 beschrieben zur Ammoniak-Rückgewinnung behandelt.

### Beispiel 7

In einer Reaktionsapparatur wurden 2,2 Mol 30%ige Salzsäure vorgelegt. Unter Rühren und Kühlen wurden 20 Minuten 2,0 Mol 97,8%iges MMP-CH zudosiert während die Temperatur auf 45 °C anstieg. Die Mischung wurde unter gelindem Kühlen bei 45 - 50 °C weitere 30 Minuten gerührt, worauf die vollständige Umsetzung des Cyanhydrins festgestellt wurde. Danach wurde in 20 Minuten auf 92 °C erhitzt und die Hydrolyse innerhalb weiterer 60 Minuten bei dieser Temperatur vervollständigt. Die im Anschluß vorgenommene Produktzerlegung und -aufarbeitung wurde wie in Beispiel 1 beschrieben vorgenommen. Nach der abschließenden Konditionierung wurden 356 g eines Konzentrates mit folgender Zusammensetzung erhalten

MHA-gesamt 80,36, davon 2,76 % freies MHA, 1,2% MHA-amid und 0,16% Dimere. Weiterhin enthielt die Lösung 8,65% NH₃, 10,9% H₂O und 1,34 Cl⁻; Der MHAAS-Gehalt betrug 84,87 %, der pH-Wert lag bei 6,4. Die Gesamtausbeute betrug 95,2 berechnet als MHA-monomer und bezogen auf eingesetztes MMP-CH. Die Lösung war nach 2monatiger Lagerzeit in ihrer Zusammensetzung unverändert.

Der bei der Reinigungsdestillation der MIBK-Phase zurückbehaltene Eindampfrückstand betrug 4,4 g und war in Wasser unlöslich.

### Beispiel 8

In einem Reaktionsgefäß gemäß Beispiel 1 wurden 134 g 30%ige Salzsäure (1,1 Mol) vorgelegt. Dazu wurde unter Rühren und Kühlen innerhalb 30 Minuten 132,5 g 99%iges MMP-CH (1,0 Mol) zugegeben, während die Temperatur auf 40 °C begrenzt wurde. Die Mischung wurde dann weitere 15 Minuten gerührt und war danach Cyanhydrin-frei. Anschließend wurde so rasch wie möglich auf 90 °C aufgeheizt und 45 Minuten lang bei dieser Temperatur hydrolysiert. Die Reaktionsmischung wurde auf ca. 30 °C (ohne Verdampfungskühlung) abgekühlt und mit 5%igem Ammoniak auf einen pH-Wert von +0,1 abgestumpft. Anschließend wurde zweimal mit je 250 ml MIBK extrahiert. Der pH-Wert der organischen Phase lag bei +0,3. Nach der Phasentrennung wurden die vereinigten Extrakte mit 1,5 Mol 20%igem Ammoniak ausgeschüttelt. Die hierbei in ihre Ammoniumsalze konvertierten MHA-Spezies wurden inklusive noch vorhandenem restlichem MHA-Amid als MHAAS und NH₄-Oligomere praktisch vollständig in die wäßrige Phase eluiert. Die leicht getrübte organische Phase wurde mit 50 ml Wasser gewaschen, worauf diese als klare Lösung erhalten wurde. Das Waschwasser wurde mit dem Eluat vereinigt und die resultierende wäßrige Phase 50 Minuten am Rückfluß erhitzt, wobei die Oligomeren wie auch MHA-amid bis auf geringe Restanteile aufgespalten wurden. Nach dem Einengen am Rotationsverdampfer wurden 170 g einer schwach gelb gefärbten Lösung vom pH-Wert 6,0 mit folgender Zusammensetzung erhalten:
MHA-gesamt 88,0 % davon 81,4 % als MHAAS und 6,6 % als freie MHA, MHA-amid 0,5 %, < 0,2 % NH₄-Dimere, 1,2 % NH₄Cl, 9,2 % NH₃ und 9,2 % H₂O. Die Gesamtausbeute betrug 99,7 %, das Konzentrat war von sehr guter Lagerstabilität.

Außerdem wurden 220 ml Raffinatlösung aus der ersten Extraktion erhalten (Primär-Raffinat), welche eine praktisch gesättigte Ammoniumchloridlösung war und eine pH-Wert von +0,15 aufwies. Diese wurde gemäß der in Beispiel 1 beschriebenen Methode mit 45%iger Natronlauge bis zu einem pH-Wert 11,8 neutralisiert. Dabei wurden ca. 90 % d.Th. des Gesamtstickstoffs in Form einer 20%igen NH₃-Lösung wiedergefunden. Das nach dem Auskochen des Raffinats erhaltene Sumpfprodukt von 225 g war eine gesättigte im wesentlichen Organik-freie und Ammoniak-freie Kochsalzlösung.

### Beispiel 9

Es wurde wie in Beispiel 8 beschrieben verfahren, jedoch wurden für die Verseifung von 1,0 Mol MMP-CH 140 g 30%ige Salzsäure (1,15 Mol) aufgewendet und die Temperatur der 1. Stufe bis auf 67 °C, die der 2. Stufe bis auf 93 °C erhöht. Das nach der Extraktion mit M IBK sowie Rückextraktion mit 20%iger Ammoniaklösung (1,5 Mol) erhaltene Eluat wurde im Vakuum schonend entgeistet. Danach wurden 202 g einer Lösung mit folgender Zusammensetzung erhalten:
MHAAS als MHA bestimmt 59,8 %, NH₄-Dimere + Trimere 10,3 % und 28,8 % H₂O. Nach der anschließenden Wärmebehandlung (45 Minuten unter Rückfluß) waren die Dimeren bis auf einen geringen Rest aufgespalten.

### Beispiel 10

Es wurde wie in Beispiel 8 beschrieben verfahren, jedoch wurden für die Verseifung 1 Mol MMP-CH und 200 g 22%ige Salzsäure (1,2 Mol) eingesetzt und die Temperatur in der 1. Stufe auf 65 °C, die der 2. Stufe auf 107 °C erhöht. Außerdem war die Reaktionszeit in der 2. Stufe um 70 Minuten verlängert. Es wurden 162 g eines braun verfärbten Konzentrates mit 84,8 % MHA-gesamt erhalten. Hiervon lagen 79,0 % als MHAAS und 5,8 % als freie MHA vor. Außerdem enthielt die Lösung 1,56 % NH₄Cl. Die Gesamtausbeute betrug 91,5 %.

### Beispiel 11

In einer kühl- wie heizbaren Reaktionsapparatur, ausgestattet mit Zulauftrichter, Intensivrührer und Rückflußkühler wurden 814,5 g 99%iges MMP-CH (6,15 Mol) mit 800 g einer 32%igen Salzsäure (7,01 Mol = 14 Mol% Überschuß) in der folgenden Weise umgesetzt:

Die Salzsäure wurde unter Eisbadkühlung vorgelegt. Das MMP-CH wurde sodann in ca. 30 Minuten zudosiert, wobei die Temperatur nicht über 40 °C anstieg und die Reaktionswärme von -91 kJ/mol MMP-CH (= -22,0 kcal) über die Außenkühlung abgeführt wurde. Anschließend wurde ohne weitere Kühlung noch 15 Minuten nachreagieren lassen, währenddessen die Temperatur auf 45 °C abstieg. Danach wurde der Reaktorinhalt mittels eines schnell heizenden Wärmebades auf 91 °C aufgeheizt und bei dieser Temperatur noch weiter 45 Minuten gerührt. In beiden Stufen wurde jeweils die Vollständigkeit des Umsatzes mittels HPLC-Analyse kontrolliert. Anschließend wurde das Heizbad entfernt und die Apparatur an ein Wasserstrahlvakuum angeschlossen. Durch adiabatische Verdampfungskühlung wurde auf ca. 60 °C abgekühlt, wobei gleichzeitig 55 g H₂O neben 14 g HCI und ca. 4 g organische Verunreinigungen abgetrieben wurden.

Nach Entspannen wurde das erhaltene Hydrolysat, welches einen pH-Wert von -0,8 aufwies, durch Zugabe von 15 g 20%iger Ammoniaklösung auf einen Wert von +0,1 neutralisiert (Vorneutralisation). Danach wurden 1545 g einer kaum verfärbten Hydrolysatlösung mit folgender Zusammensetzung erhalten:
56,5 % MHA, 1,8 % MHA-dimer + trimer, 0,4 % MHA-amid, 0,6 % HCl, 21,4 % NH₄Cl und 19,3 % H₂O.

Die Lösung wurde in eine auf ca. 40 °C temperierte und beheizte Meßvorlage überführt. Von dort wurde die Lösung in das obere Drittel einer ummantelten und auf die gleiche Temperatur gebrachte Füllkörperkolonne (80 cm Länge und 2 cm ø) mit oberer und unterer Beruhigungszone kontinuierlich mit einer Geschwindigkeit von 31 g/min aufgegeben, während im unteren Drittel mit Hilfe einer Dosierpumpe 1800 g MIBK (2245 ml) kontinuierlich mit einer Geschwindigkeit von 36 g/min eingespeist wurden. Über Kopf wurden in einer gekühlten Vorlage insgesamt 2717 g Extraktionslösung mit folgender Zusammensetzung erhalten:
66 % MIBK 33,3 % MHA-gesamt, 0,2 % MHA-amid sowie 0,2 % HCl und 0,4 % H₂O. Das ablaufende Raffinat von rd. 630 g bestand im wesentlichen aus einer übersättigten NH₄Cl Lösung von um 40 % Salzgehalt mit 0,8 % MIBK und Spuren an MHA. Es wurde unter Verdünnung auf ca. 35 % NH₄Cl-Gehalt der in Beispiel 1 beschriebene Neutralisationsapparatur zugeführt und wie dort beschrieben unter Rückgewinnung von ca. 90 % des zu erwartenden Ammoniaks aufgearbeitet.

Die Extraktionslösung wurde in eine beheizbare Rührwerksapparatur mit Rückflußkühler und bodenseitig angeschlossenem Phasentrenngefäß überführt und nach sukzessiver Zugabe von 448 g 25%igem Ammoniak ca. 10 Minuten intensiv durchmischt. Nach dem Abstellen der Rührung wurde eine kurze Beruhigungszeit von wenigen Minuten eingehalten. Danach hatten sich zwei Phasen mit scharfer Trennlinie gebildet. Nach der über den angeschlossenen Abscheider vorgenommenen Phasentrennung wurde das Eluat - die wäßrige Unterphase - in den Rührwerksbehälter zurückgegeben und 30 Minuten am Rückfluß erhitzt. Anschließend wurde Wasserstrahlvakuum angelegt und die Lösung unter Verdampfungskühlung auf 50 °C abgekühlt. Hierbei wurden der überschüssige Ammoniak von 6,8 g zusammen mit ca. 18 g Wasser abgetrieben.

### Zusammensetzung der Lösung vor der Rückflußbehandlung:

Gesamtmenge 1357 g mit 71,58 MHAAS, 2,06 % MHA-NH₄-dimer + trimer, 0,44 % MHA-amid 0,5 % NH₃, 0,65 % NH₄Cl, 24,76 % H₂O.

### Zusammensetzung des Endkonzentrates nach der Spaltung und Aufkonzentrierung:

Gesamtmenge 1182,8 g mit 84,71 % MHAAS, 0,17 % MHA-NH₄-dimer + trimer, 0,50 % MHA-amid, 0,74 % NH₄Cl, 13,87 % H₂O.

Die Gesamtausbeute berechnet als MHA und bezogen auf eingesetztes MMP-CH betrug 98,4 %. Die Lösung war von fahlgelber Farbe und hatte einen pH-Wert von 6,6. Sie war geruchsfrei, freifließend und bei 20 - 40 °C lagerstabil.

Die nach der Phasentrennung erhaltene MIBK-Oberphase wurde am Rotationsverdampfer ausdestilliert. Es wurden 99 % des eingesetzten Lösungsmittels zurückgewonnen. Außerdem erhielt man einen Eindampfrückstand von 12 g, der in Wasser unlöslich war.

### Beispiel 12

Es wurde nach der allgemeinen Verfahrensweise von Beispiel 11 ein Eluat mit 69,4 % MHAAS (monomer), 3,6 % MHA-NH₄-dimer + -trimer, 0,7 % MHA-amid und 0,7 % NH₄Cl gewonnen, das in der folgenden Weise aufgearbeitet wurde:
Die Lösung wurde in 4 gleiche Teile aufgeteilt und die vier Aliquoten einer verschieden langen Wärmebehandlung unterworfen. In Versuch (1) wurde 45 Minuten, in Versuch (2) 60 Minuten, in Versuch (3) 90 Minuten am Rückfluß erhitzt. In Versuch (4) wurde während der 60minütigen Erhitzungsdauer gasförmiger Ammoniak eingeperlt. Es wurde gefunden, daß mit zunehmender Erhitzungsdauer ach der Aufkonzentrierung bis zu einer Gesamtkonzentration von 76 % MHA entsprechend 84,7 % MHAAS unter Ammoniakverlust und pH-Erniedrigung der Anteil an freiem MHA rapide anstieg, während der Oligomeranteil auf unter 0,2 % abfiel und der MHA-amid-Anteil konstant blieb. Lediglich in Versuch (4) kam es auch zu einem Anstieg des letzteren, während umgekehrt der freie MHA-Anteil wieder zurückging. Im einzelnen wurde gefunden:
   1. Mit 45 Minuten 100 °C: 71,4 % MHAAS und 4,6 % MHA-frei, pH 6,3, lagerstabil 40 °C
   2. Mit 60 Minuten 100 °C: 66,0 % MHAAS und 10,0 % MHA-frei, pH 5,8, lagerstabil 50 °C
   3. Mit 90 Minuten 100 °C: 58,0 % MHAAS und 18,0 % MHA-frei, pH 4,9, lagerstabil 55 °C
   4. Mit 60 Minuten 100 °C: 74,0 % MHAAS und 2,0 % MHA-frei, pH 6,5, lagerstabil 35 °C + NH₃- Gas-Einperlung 2,0 % MHA-amid

### Beispiel 13

Es wurde ein Hydrolysat wie in Beispiel 6 beschrieben hergestellt und mit zweimal 300 ml MIBK = 481 g extrahiert. Es wurde eine Lösung mit 24 % MHA-gesamt erhalten, wovon 11,1 % in Form von Dimeren + Trimeren vorlagen. Die Extraktlösung wurde zur Entfernung der überschüssigen Salzsäure bis auf ein Gehalt von 30 % MHA-gesamt aufkonzentriert. Die dabei aufgetretene Trübung wurde durch Zugabe von 20 ml Wasser geklärt, worauf 1,5 Mol gasförmiger Ammoniak bei 20 °C eingeleitet wurde. Dies entsprach einer Ammoniak-Konzentration von 45,5 %. Die sich abscheidende Unterphase wurde mehrere Stunden bei 30 °C stehen gelassen und danach bei 60 °C unter Vakuum eingeengt. Es wurde ein gelblich gefärbtes Konzentrat mit einem Gehalt von 76,3 % MHA-Gesamt entsprechend 85,2 % MHAAS erhalten. Der Oligomerenanteil war bis auf < 0,2 % abgebaut, während der Anteil an MHA-amid auf 1,1 % angestiegen war. Der pH-Wert der Lösung lag um 6,5.

### Beispiel 14

Es wurde eine Reaktion wie in Beispiel 7 beschrieben durchgeführt und nach der in Beispiel 1 angegebenen allgemeinen Verfahrensweise aufgearbeitet, jedoch mit folgenden Änderungen:
Die nach der MIBK-Extraktion erhaltene 27,1%ige MHA-gesamt-Lösung wurde halbiert. In Versuch (1) wurde die Rückextraktion mit 1,1 Mol 25%igen Ammoniak, in Versuch (2) mit 0,9 Mol 25%igen Ammoniak vorgenommen. Die erhaltenen Eluate wurden anschließend 30 Minuten am Rückfluß erhitzt und danach analysiert:

### Konzentratzusammensetzung aus Versuch 1

70,4 % MHAAS, 6,2 % MHA, 0,60 % MHA-amid, 0,26 % MHA-NH₄-dimer + -trimer.

### Konzentratzusammensetzung aus Versuch 2

64,5 % MHAAS, 12,1 % MHA, 0,45 % MHA-amid, 0,46 % MHA-NH₄-dimer + -trimer.

Konzentrat (1) hatte einen pH-Wert von 6,1 und war bis ≥ 45 °C lagerstabil.

Konzentrat (2) hatte einen pH-Wert von 5,6 und war bis 60 °C lagerstabil.

### Beispiel 15

Es wurde eine MHA-Produktlösung gemäß EP 142 488, Beispiel 5 mit 89,2 % MHA-gesamt durch Hydrolyse des MMP-CH mit Schwefelsäure und nachfolgende MIBK-Extraktion nebst anschließender Wasserdampfdestillation hergestellt. Das resultierende Konzentrat hatte einen Gehalt von 72,7 % MHA-monomer und 16,5 % MHA-dimer + trimer. Diese Lösung wurde mit gasförmigem Ammoniak soweit neutralisiert, daß 78 % des MHA als Ammoniumsalz gebunden waren. Nachdem die Lösung 45 Minuten am Rückfluß erhitzt worden war, wurde das Verhältnis MHA-monomer zu MHA-oligomer mittels HPLC-Analyse erneut überprüft. Es wurde gefunden, daß der Oligomerenanteil auf 6,0 % zurückgegangen und der Monomerenanteil auf 83,2 % erhöht worden war.

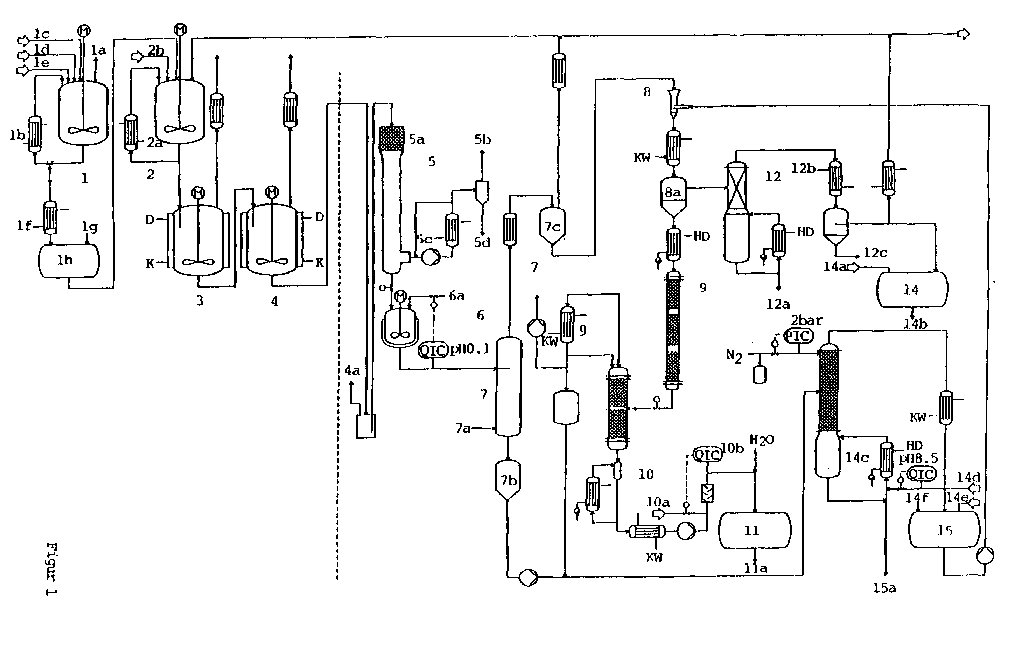

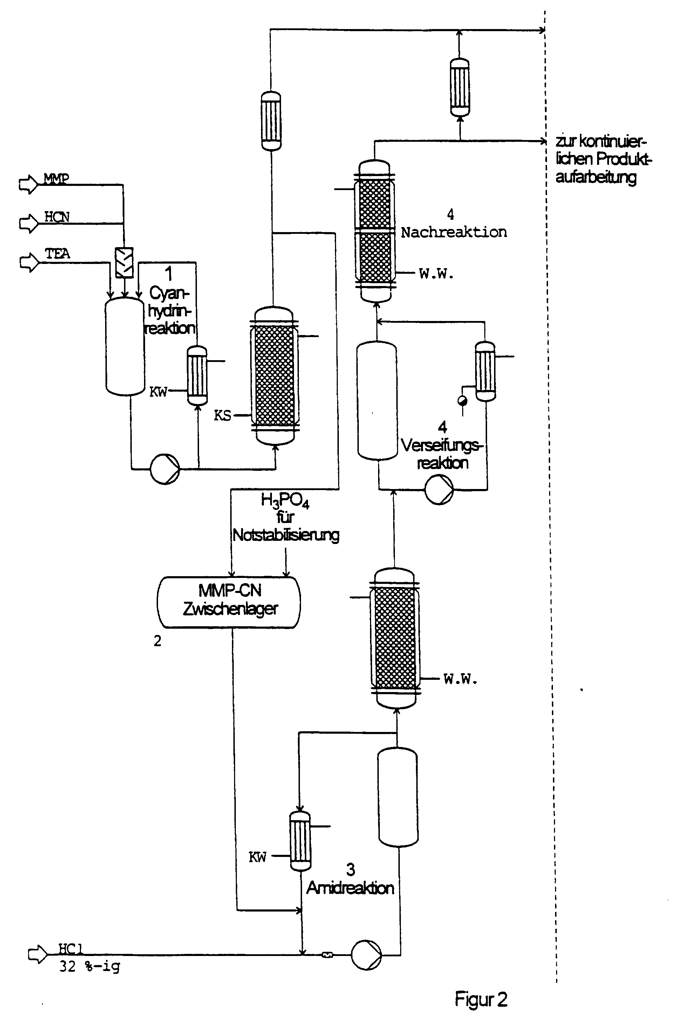

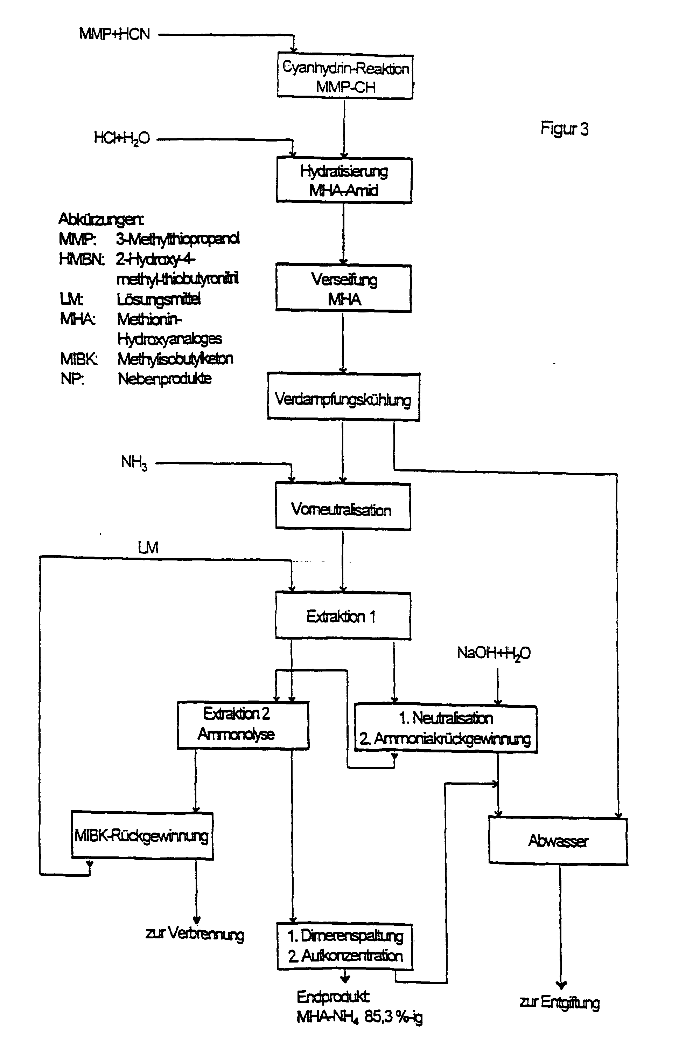

Die im Beispiel 11 beschriebene Herstellung von MHAAS ist in den Figuren 1 und 2 als technisches Verfahren mit den Hauptapparaten schematisch wiedergegeben und wird nachfolgend erläutert. Ein zugehöriges Fließdiagramm (Figur 3) zeigt nochmals den schematischen Ablauf.

### Beispiel 16

### Vorbemerkung:

In die Verfahrensbeschreibung ist die Herstellung der Cyanhydrin-Vorstufe aus den Edukten MMP und HCN einbezogen. Dementsprechend umfaßt der Reaktionsteil eine Dreistufensynthese, die sowohl ansatzweise intermittierend als auch kontinuierlich durchgeführt werden kann, während die sich anschließende Produktaufarbeitung in jedem Fall kontinuierlich erfolgt. Das im folgenden wiedergegebene Verfahren bezieht sich auf das in Figur 1 dargestellte Fließdiagramm mit diskontinuierlicher Reaktionsführung. Bei vollkontinuierlicher Arbeitsweise würde anstelle der in Figur 1 gezeigten Anordnung eine Rührkesselkaskade oder, wie in Figur 2 dargestellt, eine Batterie von 3 Schlaufenreaktoren mit nachgeschalteten Strömungsrohren als Reaktionsteil treten.

### I. Reaktion

In einem ersten Reaktor mit externem Kühlkreislauf (1) legt man MMP vor und dosiert in ca. 30 Minuten 100%ige HCN in einem geringen Überschuß von 3 Mol% bei Temperaturen um 30 °C zu, während man den pH-Wert durch Zugabe von Triethylamin (TEA) konstant auf 7,0 einreguliert und die Reaktionswärme durch Umwälzung des Gemischs über den äußeren Wärmetauscher abführt. Nach beendetem HCN-Zulauf läßt man noch ca. 30 Minuten nachreagieren und überführt das gebildete MMP-CH unter Kühlung in ein Zwischenlager¹).

In einem zweiten Reaktor mit externem Kühlkreislauf (2) legt man eine 30 - 32%ige HCl in einem 14 - 10%igen molaren Überschuß vor. Innerhalb kürzest möglicher Zeit dosiert man unter Umwälzkühlung das MMP-CH zu, während man die Temperatur bis 45 °C ansteigen läßt und dann konstant hält. Anschließend läßt man noch ca. 30 Minuten bis zum vollständigen Umsatz des Cyanhydrins nachreagieren ²⁾³⁾. Die Mischung läßt man danach in einen mit Dampf beheizten ummantelten Reaktor (3) ab und erwärmt auf 90 - max. 93 °C. Nach einer Reaktionsdauer von 45 Minuten überführt man die Mischung zur Vervollständigung der Hydrolyse in einen ummantelten und beheizten Reaktor (4), der sowohl als Nachreaktor als auch als Pufferbehälter dient und in welchem das Hydrolysat nach analytischer Kontrolle für die anschließende kontinuierliche Produktaufarbeitung vorgehalten wird.

### II. Kontinuierliche Produktaufarbeitung

Die im Nachreaktor/Pufferbehälter (4) intermittierend akkumulierte und entgeistete Hydrolyselösung wird über eine barometrische Abtauchung einem unter Vakuum stehenden, mit zugehöriger Peripherie ausgestattetem Fallfilmverdampfer (5) aufgegeben. Dabei kühlt sich der ablaufende Produktstrom infolge adiabatischer Verdampfungskühlung auf ca. 60 °C ab, während flüchtige Verunreinigungen zusammen mit anteiligen Mengen an Wasser und Salzsäure abgetrieben werden. Die resultierenden Brüden werden nach Partialkühlung noch gequencht, wobei die Majorität der überschüssigen HCl als wäßrige Lösung auskondensiert wird⁴) und das verbleibende Abgas einem Inzinerator zwecks Verbrennung zugeführt wird.

Die entgeistete Hydrolyselösung gelangt unter Entspannung in einen aus einen ummantelten, kühl- wie heizbaren sowie mit Mess- und Regel-Einrichtungen für pH-Wert, Temperatur und Niveau ausgestatteten Rührwerksbehälter (6), in welchem sie mit konz. Ammoniaklösung bei 40 - 50 °C auf einen pH-Wert um +0,1 abgestumpft wird.

Der aus (6) ablaufende Produktstrom wird in den oberen Teil einer mit zugehöriger Peripherie bestückten Extraktionskolonne (7) eingespeist, während man in Bodennähe MIBK (oder einem anderen geeigneten Lösungsmittel) im Gegenstrom in einen Aufnehmer/Abnehmer-Verhältnis von ca. 1,1 - 1,2 : 1 diesem entgegenleitet. Der resultierende nunmehr zweigeteilte Produktstrom wird wie folgt weiter aufgearbeitet:
Der über Kopf unter Kühlung abgenommene Extrakt wird über einen Puffer in einen Saugstrahler (8) eingesaugt und dort mit ca. 20%iger, aus der Neutralisationsstufe zurückgewonnener Ammoniaklösung (s. w. u.) in geregeltem Überschuß von 1 : 1,1 bis 1,3 Mol vermischt. Dabei erfolgt die Neutralisation des MHA zu MHAAS sowie der entsprechenden Oligomeren-Spezies zu deren Ammoniumsalzen nebst deren Rückextraktion in eine wäßrige Phase. Das nach der MHA/MHAAS-Konvertierung unter Kühlung ablaufende Gemisch wird in einem nachgeschalteten zu (8) gehörigen Abscheider aufgenommen und unter Phasentrennung weiterverarbeitet.
Die organische Oberphase wird in eine mit entsprechender Peripherie bestückte Destillationsanlage (12) eingespeist. Das hierin destillativ gereinigte und zurückgewonnene MIBK wird nach Passieren eines Abscheiders in ein Lösungsmittellager (16) abgeführt, von wo es in den primären Extraktionskreislauf rezykliert wird. Die im Abscheider zurückgehaltenen Wasserreste werden über einen Sammler in den Destillationskreislauf zurückgeführt. Das ausgeschleuste Sumpfprodukt wird verbrannt.

Die wäßrige Unterphase - das Eluat - wird über einen Vorerhitzer in eine aus zwei Hauptapparaten bestehende Spalt/Konzentrationsanlage (9) abgelassen. Dabei durchläuft die Lösung zunächst eine auf 90 °C temperierte Verweilzeitkolonne und gelangt anschließend in eine unter schwachem Vakuum und mit Rückfluß betriebene Destillationskolonne. Hierbei erfolgt die Spaltung der Oligomerenanteile als auch gleichzeitig Aufkonzentrierung unter Entfernung von überschüssigem Ammoniak⁵), welcher als Brüdenprodukt mit dem Raffinatstrom zur Ammoniakrückgewinnung (s.w.u.) vereinigt wird. Das aus dem Sumpf unter Kühlung und Entspannung ablaufende Konzentrat durchläuft noch eine mit Mischer- sowie mit entsprechenden Dosier- und M+R-Einrichtungen bestückte Konditionierungsstrecke (10), in der die gewünschte Produktspezifikation durch wahlweise Zugabe von Ammoniak⁵) und/oder Wasser eingestellt wird. Das konditionierte Endprodukt wird anschließend in das Fertigproduktlager (11) übernommen.

Das aus der Extraktionskolonne (7) ablaufende, im wesentlichen aus einer ge- bis übersättigten Ammoniumchloridlösung⁶) bestehende Raffinat wird in eine unter geringem Überdruck betriebene Destillationskolonne (14) eingespeist, während über den zugehörigen Heizkreislauf der Anlage pH-geregelt konzentrierte Natronlauge zugemischt wird. Dabei wird das Ammonsalz vollständig zu Natriumchlorid und Ammoniak umgesetzt. Der freigesetzte Ammoniak wird gleichzeitig vollständig bei pH-Werten > 8 ausdestilliert und nach der Kondensation als 20 - 25%ige wäßrige Lösung in ein NH₃-Lager (15) überführt und dort für die Rückextraktion und Neutralisation des MHA unter MHAAS-Bildung vorgehalten⁶). Der im wesentlichen aus einer gesättigten Kochsalzlösung bestehende Sumpfablauf wird ggf. unter Nachneutralisation mit Salzsäure⁴) nach analytischer Kontrolle⁷) an eine biologische Abwasserreinigungsanlage abgegeben.

### Anmerkungen:

1) Für den Fall, daß MMP-CH längere Zeit und/oder ungekühlt zwischengelagert werden muß, ist eine mit entsprechenden Zusatzeinrichtungen ausgestattete Notstabilisierung, beispielsweise mit Phosphorsäure, vorzusehen. Dazu ist erforderlich, daß der Lagerinhalt entweder mittels Rührer oder mittels Pumpenumwälzung durchmischt wird (im Schema nicht eingezeichnet)
2) Der molare HCI-Überschuß hängt von der Konzentration der Säure ab und ist um so niedriger, je höher letztere ist.
3) Ein geringer Anteil des MHA-amids wird bereits in der Amidierungsstufe zum MHA weiter hydrolysiert.
4) Die zurückgewonnene wäßrige HCI kann bei Bedarf für die Neutralisation des Abwassers verwendet werden. Andernfalls wird sie als Prozeßsäure rezykliert.
5) Bei der Oligomerenspaltung kann es in gewissem Umfang in Abhängigkeit von der Verweilzeit und der Temperatur durch Hydrolyse unter NH₃-Ausgasung zur Rückbildung von MHA kommen. Übersteigt der MHA-Anteil die gewünschte Spezifikationsgrenze z.B. von 5 bis max. 10 Mol%, so wird der Überschußanteil in der Konditionierungsstrecke durch pH-geregelte NH₃-Zugabe nachneutralisiert. Wichtig hierbei ist, daß es zu keiner nennenswerten Rückbildung von Oligomeren kommt.
6) Der Raffinatstrom ist in Abhängigkeit von dem gewählten Lösungsmittel - hier mit MIBK - kontaminiert. Dieses findet sich dann in dem entgeisteten und rekondensierten Kreislauf-Ammoniak wieder. Um eine Akkumulierung zu verhindern, wobei Entmischung erfolgt, muß der NH₃-Kondensation ein Separator nachgeschaltet werden (im Schema nicht eingezeichnet).
7) Analytisch überwacht wird neben den charakteristischen Abwasserwerten wie BSB, TOC, AOX vor allem ein etwaiger Restcyanidgehalt (aus dem Brüdenkondensat der Verdampfungskühlung). Überschreitet dieser den zulässigen Grenzwert, so muß er durch Zumischung von Wasserstoffperoxid, beispielsweise in den Heizkreislauf von (14), entsprechend abgesenkt werden.

### Beispiel 17

### Vergleichende Nutrivitätsuntersuchungen

In vergleichenden Broilerwachstumstest wurde an 7 Tage alten Küken über 21 Tage die biologische Wirksamkeit der Methioninanalogen MHA und MHAAS untersucht. Als Kriterien dienten der Gewichtszuwachs und die Futterverwertung der 7 - 28 Tage alten Tiere. Ausgangspunkt war eine S-aminosäurearme Basalration, deren M + C-Gehalt von 0,40 % auf 0,68 % angehoben wurde. Hierbei wurden die Methioninsubstitute äquimolar in den folgenden 8 Supplementierungsstufen zugelegt (in Prozenten):

0,02 - 0,04 - 0,07 - 0,10 - 0,14 - 0,18 - 0,23 - 0,28.

### A. Zusammensetzung der Basalration

| Componenten % | | Aminosäuren % | |
|---|---|---|---|
| 20,0 | Mais | M + C : 0,40 | Arg : 1,35 |
| 11,6 | Maisstärke | Met : 0,19 | Ile : 0,80 |
| 29,93 | Tapioca | Cys : 0,21 | Val : 1,00 |
| 5,5 | Sojaöl | Lys : 1,15 | Leu : 1,35 |
| 5,5 | Cellulose | Thr : 0,80 | Trp : 0,22 |
| 20,0 | Sojamehl | | |
| 0,73 | CaCO₃ | Na : | Ca : 0,95 |
| 2,2 | CaHPO₄ x 2H₂O | | P : 0,65 |
| | | C. P. : | 16,0 |
| Energie (MJ/kg) ME-Küken 13,6 | | | |

### B. Versuchsbedingungen

7 Tage alte Broiler (Küken)
Zahl der Tiere: 1125
Wiederholungen: 3
Versuchsdauer: 21 Tage

### C. Versuchsergebnisse (s. 2 - 4)

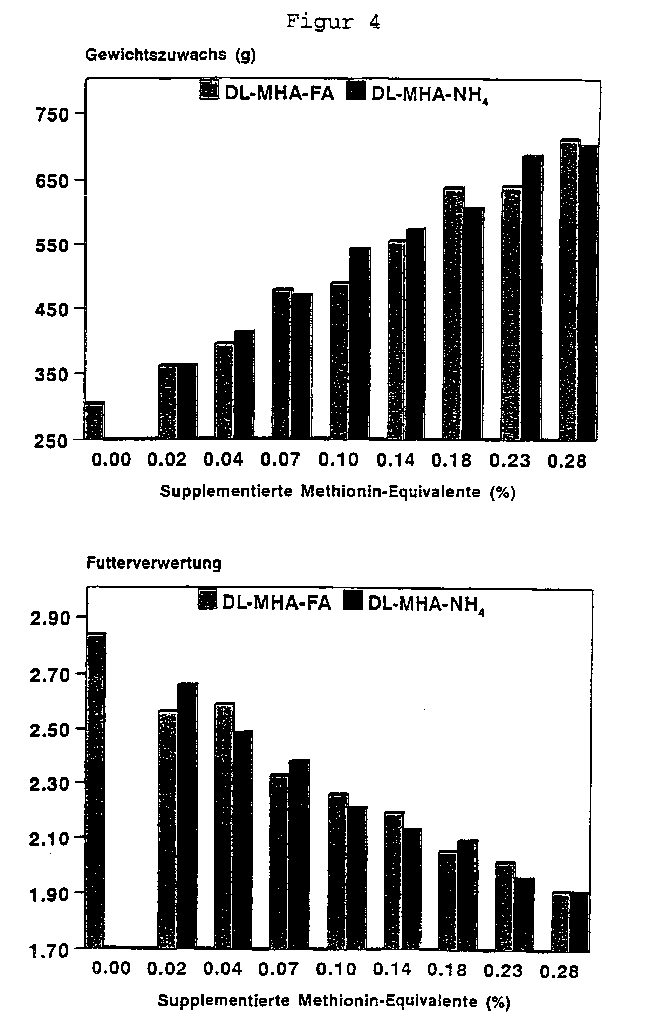

Die in den einzelnen Behandlungsgruppen erzielten Leistungen sind der Ergebnistabelle 1 und den grafischen Darstellungen (Figuren 4 und 5) zu entnehmen.

**Tabelle 1**

| Ergebnisse: 7 - 28 Tage | | | | | |
|---|---|---|---|---|---|
| **Gruppe** | **Produkt** | **Dosis %** | **Gewichts- zuwachs g** | **Futter- umsatz** | **berechnete Futter- aufnahme g** |
| 01 | Kontroll | 0,000 | 303 | 2,84 | 861 |
| 10 | DL-MHA (freie Säure) | 0,020 | 361 | 2,57 | 927 |
| 11 | DL-MHA (freie Säure) | 0,040 | 395 | 2,59 | 1024 |
| 12 | DL-MHA (freie Säure) | 0,070 | 478 | 2,33 | 1112 |
| 13 | DL-MHA (freie Säure) | 0,100 | 489 | 2,26 | 1105 |
| 14 | DL-MHA (freie Säure) | 0,140 | 554 | 2,19 | 1215 |
| 15 | DL-MHA (freie Säure) | 0,180 | 636 | 2,05 | 1306 |
| 16 | DL-MHA (freie Säure) | 0,230 | 640 | 2,01 | 1288 |
| 17 | DL-MHA (freie Säure) | 0,280 | 712 | 1,91 | 1360 |
| 18 | DL-MHA-NH₄ | 0,020 | 362 | 2,66 | 964 |
| 19 | DL-MHA-NH₄ | 0,040 | 413 | 2,49 | 1027 |
| 20 | DL-MHA-NH₄ | 0,070 | 469 | 2,38 | 1116 |
| 21 | DL-MHA-NH₄ | 0,100 | 542 | 2,21 | 1198 |
| 22 | DL-MHA-NH₄ | 0,140 | 572 | 2,13 | 1220 |
| 23 | DL-MHA-NH₄ | 0,180 | 605 | 2,09 | 1266 |
| 24 | DL-MHA-NH₄ | 0,230 | 685 | 1,96 | 1341 |
| 25 | DL-MHA-NH₄ | 0,280 | 703 | 1,91 | 1343 |

Sowohl beim Gewichtszuwachs als auch bei der Futterverwertung verbesserten sich die Leistungen der Tiere kontinuierlich mit steigender Dosierung der Methioninquellen.
Mit der höchsten Supplementierung konnte gegenüber der unsupplementierten Kontrollgruppe eine deutliche Gewichtssteigerung und eine Verbesserung der Futterverwertung erzielt werden. Die Versuchsdaten ließen insgesamt einen guten Response auf die zugelegten Substanzen erkennen.
Die durchschnittliche Gewichtszunahme in den einzelnen Behandlungsgruppen gegenüber der unsupplementierten Kontrollgruppe betrug beim MHA 230 g und beim MHAAS 241 g. Hieraus ergibt sich ein Wertigkeitsfaktor von 95,6 % beim MHA gegenüber dem MHAAS

### D. Biologische Wertigkeit der beiden Methioninanalogen

Für D,L-MHA berechnete sich eine durchschnittliche Wertigkeit von 93,3 % bezogen auf MHAAS. Beide Methioninanaloga werden zwar deutlich schlechter verwertet als D,L-Methionin, jedoch zeigte das MHAAS eine ausgeprägte, nämlich um rd. 6 % höhere biologische Wertigkeit.

Weitere Einzelheiten und Vorteile der Erfindung ergeben sich aus den nachfolgenden Patentansprüchen.

### Bezugszeichenliste zu Fig. 1

- 1: Cyanhydrinreaktion
- 1a: z. Wäsche
- 1b: KW
- 1c: MMP
- 1d: HCN
- 1e: TEA
- 1f: KS
- 1g: H₃PO₄ für Notstabilisierung
- 1h: MMP-CN-Zwischenlager
- 2: Amidreaktion
- 2a: KW
- 2b: HCl 32 %ig
- 3: Verseifung
- 4: Nachreaktion und Puffer
- 4a: z. Abluftwäscher
- 5: Verdampfungskühlung
- 5a: Fallfilmverdampfer
- 5b: zum Inzinerator
- 5c: KW
- 5d: Abwasser z. Biologie
- 6: Vorneutralisation
- 6a: NH₃ v. Lager 15
- 7: Extraktion I
- 7a: MiBK v. Lager 14
- 7b: Puffer
- 7c: Puffer
- 8: Ammonolyse + Extraktion II
- 8a: Abscheider
- 9: Dimerenspaltung
- 10: Konditionierung
- 10a: Frisch NH₃
- 10b: pH 7,0
- 11: Lager MHA-NH₄ ~85 %ig
- 11a: z. Abfüllung
- 12: MiBK-Destillation
- 12a: Ausschleusung zum Inzinerator
- 12b: KW
- 12c: zum Sammler 16
- 14: MiBK-Lager
- 14a: Frisch MiBK
- 14b: MiBK z. Extraktion I
- 14c: Neutralisation
- 14d: NaOH 50 %ig
- 14e: Frisch NH₃
- 14d: H₂O
- 15: NH₃-Lager ∼30 %ig
- 15a: Abwasser z. Biologie

## Patentansprüche

1. Verfahren zur Herstellung von Ammonium-2-hydroxy-4-methylthio-n-butyrat (MHAAS) durch Behandlung von 2-Hydroxy-4-methylthiobuttersäure (MHA) mit Ammoniak, wobei MHA auch Dimere und höhere Oligomere umfaßt und aus einem Reaktionsgemisch isoliert wird, welches durch Anlagerung von Blausäure (HCN) an Methylmercaptopropionaldehyd (MMP) und Hydrolyse mit HCl des dabei erhaltenen Methylmercaptopropionaldehyd-Cyanhydrins (MMP-CH) erhalten wird,
**dadurch gekennzeichnet,**
**daß** das Reaktionsgemisch mit einem inerten, mit Wasser nicht oder nur teilweise mischbaren Lösungsmittel unter Erhalt eines ersten organischen Extrakts und eines ersten wäßrigen Raffinats behandelt wird, der erste organische Extrakt durch Behandlung mit Ammoniak unter Phasenseparierung in einen zweiten organischen Extrakt und ein zweites wäßriges Raffinat zerlegt wird, wobei unter Salzbildung die Rückextraktion des MHA als MHAAS in das zweite wäßrige Raffinat erfolgt, und das MHAAS aus dem zweiten wäßrigen Raffinat isoliert wird.

2. Verfahren nach Anspruch 1,
**dadurch gekennzeichnet,**
**daß** der erste organischen Extrakt so und in solcher Menge mit konzentrierter, vorzugsweiser 25 - 90 gewichtsprozentiger wäßriger Ammoniaklösung versetzt wird, daß das darin enthaltene MHA im wesentlichen vollständig zum MHAAS neutralisiert wird.

3. Verfahren nach einem der vorangehenden Ansprüche,
**dadurch gekennzeichnet,**
**daß** das zweite wäßrige Raffinat vor und/oder während der Isolierung des MHAAS erhitzt wird, vorzugsweise unter Rückfluß.

4. Verfahren nach einem der vorherigen Ansprüche,
**dadurch gekennzeichnet,**
**daß** das zweite Raffinat zur Isolierung des MHAAS unter Abdampfen von freiem ggf. überschüssigem Ammoniak durch Eindampfen aufkonzentriert wird.

5. Verfahren nach einem der Ansprüche 3 oder 4,
**dadurch gekennzeichnet,**
**daß** das zweite Raffinat so erhitzt wird, daß die dimeren und höheren oligomeren Anteile des MHAAS sowie ggf. noch restliches MHA-Amid bis zum gewünschten Grenzgehalt unter Bildung von weiterem monomerem MHAAS gespalten werden.

6. Verfahren nach einem der Ansprüche 3 - 5,
**dadurch gekennzeichnet,**
**daß** das zweite Raffinat so erhitzt wird, daß eine ggf. erwünschte Menge an MHA durch hydrolytische Spaltung des MHAAS unter Freisetzung von Ammoniak rückgebildet wird.

7. Verfahren nach einem der vorhergehenden Ansprüche,
**dadurch gekennzeichnet,**
**daß** im zweiten Raffinat ggf. vorhandener überschüssiger Ammoniak zur Isolierung des MHAAS abgedampft wird in Verbindung mit einer Aufkonzentrierung durch Wasserverdampfung mittels einer adiabatischen Verdampfungskühlung.

8. Verfahren nach Anspruch 1,
**dadurch gekennzeichnet,**
**daß** die Hydrolyse des MMP-CH in zwei Stufen durchgeführt wird, wobei in einer ersten Stufe wäßrige Salzsäure mit einer Konzentration von 15 - 40 Gewichtsprozent in einem Temperaturbereich von 20 - 70 °C in einem Molverhältnis MMP-CH : HCl wie 1 : 1,0 bis 1 : 1,5 verwendet wird.

9. Verfahren nach Anspruch 8,
**dadurch gekennzeichnet,**
**daß** HCl in einer solchen Menge und unter solchen Bedingungen zugegeben wird, daß nach der ersten Hydrolysestufe aus dem MMP-CH im wesentlichen MHA-Amid entsteht und die dabei entstandene Reaktionsmischung Cyanhydrin-frei ist.

10. Verfahren nach einem der Ansprüche 8 oder 9,
**dadurch gekennzeichnet,**
**daß** die nach der ersten Hydrolysestufe erhaltene Reaktionsmischung in einer zweiten Stufe, ggf. nach vorheriger Verdünnung mit Wasser bei Temperaturen von 80 - 110 °C , vorzugsweise von 90 - 95 °C, gehalten wird, um die Hydrolyse des MHA-Amids zum MHA zu vervollständigen.

11. Verfahren nach einem der vorangehenden Ansprüche,
**dadurch gekennzeichnet,**
**daß** das nach der sauren Hydrolyse erhaltene Reaktionsgemisch unter Anwendung einer adiabatischen Verdampfungskühlung zur Entfernung geringer Mengen flüchtiger Verbindungen auf Temperaturen ≤ 70 °C, bevorzugt ≤ 60 °C, gekühlt wird.

12. Verfahren nach einem der vorangehenden Ansprüche,
**dadurch gekennzeichnet,**
**daß** das Reaktionsgemisch nach der Hydrolyse und vor der Behandlung mit dem Lösungsmittel mit einer Base neutralisiert wird.

13. Verfahren nach Anspruch 12,
**dadurch gekennzeichnet,**
**daß** mit vorzugsweise gasförmigem Ammoniak auf einen pH-Wert im Bereich von -0,5 bis +0,5 eingestellt wird.

14. Verfahren nach einem der Ansprüche 12 oder 13, soweit diese auf einen der Ansprüche 8 bis 11 zurückbezogen sind,
**dadurch gekennzeichnet,**
**daß** das im wesentlichen Ammoniumchlorid enthaltende Raffinat mit Natronlauge bis zu pH-Werten ≥ 8 unter Freisetzung von Ammoniak und Bildung einer mit Ammoniak gesättigten NaCl-Lösung versetzt wird.

15. Verfahren nach wenigstens einem der Ansprüche 7 und 14,
**dadurch gekennzeichnet,**
**daß** der freigesetzte Ammoniak abdestilliert, ggf. aufkonzentriert und zur Behandlung des zweiten wäßrigen Raffinats rezykliert wird.

## Claims

1. Process for the preparation of ammonium 2-hydroxy-4-methylthio-n-butyrate (MHAAS) by treating 2-hydroxy-4-methylthiobutyric acid (MHA) with ammonia, the MHA also containing dimers and higher oligomers and being isolated from a reaction mixture which is obtained by addition of hydrocyanic acid (HCN) to methylmercaptopropionaldehyde (MMP) and hydrolysis with hydrochloric acid of the methylmercaptopropionaldehyde cyanohydrin (MMP-CH) thus obtained,
**characterised in that**
the reaction mixture is treated with an inert solvent which is immiscible or only partially miscible with water, a first organic extract and a first aqueous raffinate being obtained; by treatment with ammonia the first organic extract is split up, with phase separation, into a second organic extract and a second aqueous raffinate, the reextraction of the MHA as MHAAS into the second aqueous raffinate taking place accompanied by salt formation, and the MHAAS is isolated from the second aqueous raffinate.

2. Process according to claim 1,
**characterised in that**
concentrated, preferably 25 to 90 per cent by weight aqueous ammonia solution is added to the first organic extract in such a manner and in such a quantity that the MHA present in the latter is substantially completely neutralised to MHAAS.

3. Process according to one of the preceding claims,
**characterised in that**
the second aqueous raffinate is heated, preferably under reflux, before and/or during the isolation of MHAAS.

4. Process according to one of the preceding claims,
**characterised in that**
the second aqueous raffinate is concentrated by evaporation, with evaporation off of free, optionally excess ammonia, in order to isolate MHAAS.

5. Process according to one of claims 3 or 4,
**characterised in that**
the second raffinate is heated so that the dimeric and higher oligomeric contents of the MHAAS and any residual MHA amide are decomposed until the required limiting content is attained, with the formation of further monomeric MHAAS.

6. Process according to one of claims 3 to 5,
**characterised in that**
the second raffinate is heated so that an optionally desired quantity of MHA is formed again by hydrolytic decomposition of MHAAS, with release of ammonia.

7. Process according to one of the preceding claims,
**characterised in that**
any excess ammonia present in the second raffinate is evaporated off, in conjunction with a concentration by steam vaporisation by means of an adiabatic evaporative cooling, in order to isolate MHAAS.

8. Process according to claim 1,
**characterised in that**
the hydrolysis of MMP-CH is carried out in two steps, aqueous hydrochloric acid being used in a first step at a concentration of from 15 to 40 per cent by weight within a temperature range of from 20°C to 70°C and in a molar ratio of MMP-CH : HCl such as 1 : 1.0 to 1 : 1.5.

9. Process according to claim 8,
**characterised in that**
HCl is added in such a quantity and under such conditions that, after the first hydrolysis step, substantially MHA amide is formed from the MMP-CH and the reaction mixture thus obtained is free from cyanohydrin.

10. Process according to one of claims 8 or 9,
**characterised in that**
in a second step, the reaction mixture obtained after the first hydrolysis step, optionally after previous dilution with water, is maintained at temperatures of from 80°C to 110°C and preferably from 90°C to 95°C, in order to complete the hydrolysis of MHA amide to MHA.

11. Process according to one of the preceding claims,
**characterised in that**
the reaction mixture obtained after the acid hydrolysis is cooled to temperatures of ≤ 70°C and preferably of ≤ 60°C, with application of an adiabatic evaporative cooling, in order to remove small quantities of volatile compounds.

12. Process according to one of the preceding claims,
**characterised in that**
after the hydrolysis and before the treatment with the solvent, the reaction mixture is neutralised with a base.

13. Process according to claim 12,
**characterised in that**
the pH value is adjusted within a range of -0.5 to +0.5, using preferably gaseous ammonia.

14. Process according to one of claims 12 or 13, in so far as these are related back to one of claims 8 to 11,
**characterised in that**
sodium hydroxide solution is added to the raffinate, which contains substantially ammonium chloride, until pH values of ≥ 8 are attained, with release of ammonia and formation of an NaCl solution saturated with ammonia.

15. Process according to at least one of claims 7 and 14,
**characterised in that**
the ammonia released is distilled off, optionally concentrated and recirculated for the treatment of the second aqueous raffinate.

## Revendications

1. Procédé de préparation de 2-hydroxy-4-méthylthio-n-butyrate d'ammonium (MHAAS) par traitement d'acide 2-hydroxy-4-méthylthiobutyrique (MHA) avec de l'ammoniac, le MHA comprenant également des dimères et des oligomères supérieurs et étant isolé d'un mélange réactionnel qui est obtenu par addition d'acide cyanhydrique (HCN) à un mercaptopropionaldéhyde de méthyle (MMP) et hydrolyse, avec HCl de la cyanhydrine de mercaptopropionaldéhyde de méthyle aindi obtenue (MMP-CH),
**caractérisé en ce que**
le mélange réactionnel est traité avec un solvant inerte non miscible ou seulement partiellement miscible à l'eau pour obtenir un premier extrait organique et un premier raffinat auqueux, le premier extrait organique est décomposé, par traitement avec de l'ammoniac, avec séparation en phases, en un second extrait organique et
en un second raffinat aqueux, où, par formation de sels, la réextraction du MHA sous forme de MHAAS se fait dans le second raffinat aqueux et le MHAAS est isolé du second raffinat auqueux.

2. Procédé selon la revendication 1,
**caractérisé en ce**
**qu'**on fait réagir le premier extrait organique en quantité telle, avec une solution d'ammoniac concentrée aquese de préférence à 25 à 90% en poids, que le MHA qui s'y trouve est neutralisé sensiblement complètement en MHAAS.

3. Procédé selon l'une quelconque des revendications précédentes,
**caractérisé en ce que**
le second raffinat aqueux est chauffé avant et/ou pendant l'isolement du MHAAS, de préférence au reflux.

4. Procédé selon l'une quelconque des revendications précédentes,
**caractérisé en ce que**
le second raffinat utilisé pour isoler le MHAAS par évaporation de l'ammoniac libre ou excédentaire.est concentré par évaporation

5. Procédé selon l'une quelconque des revendications 3 et 4,
**caractérisé en ce que**
le second raffinat est chauffé de telle sorte que les fractions dimères et oligomères supérieures du MHAAS ainsi qu'éventuellement le MHA-amide qui reste encore soient décomposés jusqu'à la teneur limite souhaitée en formant une quantité supplémentaire de MHAAS monomère.

6. Procédé selon l'une quelconque des revendications 3 à 5,
**caractérisé en ce que**
le second raffinat est chauffé de telle sorte qu'une quantité éventuellement souhaitée de MHA soit reformée par décomposition hydrolytique du MHAAS en libérant de l'ammoniac.

7. Procédé selon l'une quelconque des revendications précédentes,
**caractérisé en ce que**
l'ammoniac excédentaire éventuellement présent dans le second raffinat est évaporé pour isoler le MHAAS en liaison avec une concentration par évaporation de l'eau au moyen d'un refroidissement par évaporation.

8. Procédé selon la revendication 1,
**caractérisé en ce que**
l'hydrolyse du MMP-CH se fait en deux étapes, en utilisant, dans une première étape, de l'acide chlorhydrique aqueux en concentration de 15 à 40% en poids dans une plage de températures de 20 à 70°C et dans un rapport molaire MMP-CH:HCl tel que 1:1,0 à 1:1,5.

9. Procédé selon la revendication 8,
**caractérisé en ce**
**qu'**on ajoute du HCl en quantité et dans des conditions telles qu'après la première étape d'hydrolyse, on obtienne à partir du MMP-CH en substance du MHA-amide et que le mélange réactionnel ainsi produit soit exempt de cyanhydrine.

10. Procédé selon l'une quelconque des revendications 8 et 9,
**caractérisé en ce que**
le mélange réactionnel obtenu après la première étape d'hydrolyse est maintenu, dans une seconde étape, éventuellement après dilution préalable avec de l'eau, à des températures de 80 à 110°C, de préférence de 90 à 95°C, pour compléter l'hydrolyse du MHA-amide en MHA.

11. Procédé selon l'une quelconque des revendications précédentes,
**caractérisé en ce que**
le mélange réactionnel obtenu après l'hydrolyse acide est refroidi à des températures inférieures ou égales à 70°C, de préférence inférieures ou égales à 60°C, en utilisant un refroidissement adiabatique par évaporation pour éliminer les faibles quantités de composés volatils.

12. Procédé selon l'une quelconque des revendications précédentes,
**caractérisé en ce que**
le mélange réactionnel est neutralisé avec une base après l'hydrolyse et avant le traitement avec le solvant.

13. Procédé selon la revendication 12,
**caractérisé en ce**
**qu'**on règle la valeur du pH dans la plage de, -0,5 à +0,5 avec de l'ammoniac de préférence gazeux.

14. Procédé selon l'une quelconque des revendications 12 et 13, dans la mesure où elles se réfèrent aux revendications 8 à 11,
**caractérisé en ce**
**qu'**on fait réagir le raffinat contenant en substance du chlorure d'ammonium avec une lessive sodique jusqu'à des valeurs de pH supérieures ou égales à 8 en libérant de l'ammoniac et en formant une solution de NaCl saturée d'ammoniac.

15. Procédé selon au moins l'une quelconque des revendications 7 et 14,
**caractérisé en ce que**
l'ammoniac libéré est séparé par distillation, éventuellement concentré et recyclé pour le traitement du second raffinat aqueux.
